(19) Europäisches Patentamt
European Patent Office
Office européen des brevets

(11) EP 4 000 725 A1

(12) **EUROPEAN PATENT APPLICATION**

(43) Date of publication:
**25.05.2022 Bulletin 2022/21**

(21) Application number: **21208928.8**

(22) Date of filing: **18.11.2021**

(51) International Patent Classification (IPC):
***B01J 13/14*** (2006.01) ***A01N 25/28*** (2006.01)
***A61K 8/11*** (2006.01) ***A61K 9/50*** (2006.01)
***B01J 13/16*** (2006.01) ***B01J 13/22*** (2006.01)
***C09B 67/02*** (2006.01) ***C11D 3/50*** (2006.01)
***D06M 23/12*** (2006.01) ***F28D 20/02*** (2006.01)

(52) Cooperative Patent Classification (CPC):
**B01J 13/14; A61K 8/11; A61K 8/85; A61Q 13/00; B01J 13/16; B01J 13/22; C09B 67/0097; C11D 3/505; D06M 23/12**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **19.11.2020 US 202063116013 P**

(71) Applicant: **The Procter & Gamble Company**
**Cincinnati, OH 45202 (US)**

(72) Inventors:
- **Fernandez Prieto, Susana**
  **1853 Strombeek-Bever, Brussels (BE)**
- **Smets, Johan**
  **1853 Strombeek-Bever, Brussels (BE)**
- **Feng, Linsheng**
  **Appelton / WI, 54915 (US)**
- **Bardsley, Travis Ian**
  **Appelton / WI, 54915 (US)**
- **Chakar, Fadi Selim**
  **Appelton / WI, 54915 (US)**
- **Bobnock, Robert Stanley**
  **Appelton, 54915 (US)**
- **De Nies, Peter**
  **1853 Strombeek-Bever, Brussels (BE)**

(74) Representative: **Hoyng Rokh Monegier B.V.**
**Rembrandt Tower, 30th Floor**
**Amstelplein 1**
**1096 HA Amsterdam (NL)**

(54) **CONSUMER PRODUCT COMPRISING POLY ACRYLATE AND POLY(BETA-AMINO ESTER) DELIVERY CAPSULES WITH ENHANCED DEGRADABILITY**

(57) Consumer product composition having delivery particles encapsulating oily core materials have a shell material of hybrid poly acrylate and poly(beta-amino esters) (PAC/PBAE). The delivery particles may have a single shell of hybrid PAC/PBAE, dual shells including hybrid PAC/PBAE in an inner shell and PBAE in an outer shell crosslinked to the inner shell, or multiple shells including PAC in an inner shell, hybrid PAC/PBAE in a transitioning shell, and PBAE in an outer shell. Formation of the delivery particles includes polymerization between multifunctional amine and multifunctional acrylate to produce a water-soluble PBAE; polymerization between the preformed PBAE prepolymer having free methacrylate moieties reactive with a multifunctional (meth)acrylate in the oil phase, or at an interface of the water and oil phases to produce PAC wall, polymerization between polyacrylate and the amine moiety of PBAE prepolymer to produce hybrid PAC/PBAE delivery particle wall; and polymerization between multifunctional acrylate and primary or secondary amine moiety of the PBAE prepolymer to form a PBAE outer shell.

EP 4 000 725 A1

## Description

FIELD OF DISCLOSURE

The present invention relates to compositions comprising encapsulation compositions for encapsulating active material and processes for making such encapsulation compositions, and in particular to an encapsulation composition including the polymeric shell of the capsules comprising a hybrid polymer of poly acrylate and poly(beta-amino esters) (PAC/PBAE) and encapsulating oil phase active material.

BACKGROUND OF THE INVENTION

Microencapsulation is a process of building a functional barrier between the core material and the surrounding material to avoid chemical and physical reactions and to maintain the biological, functional, and physicochemical properties of core material. Microencapsulating particulate materials are of interest where there is a need to deliver, apply, or release an active material including, for example, a fragrance, flavor, and pesticide, to a target area in a time-delayed or controlled manner.

In view of the many uses for microencapsulation in many diverse fields, it is desirable to provide a microencapsulation composition with improved biodegradability and encapsulation performance at the same time, which would allow for protection against unwanted degradation, targeting specific and controlled release of the active substance, facilitating increased efficacy and availability, and removal from the body via normal metabolic pathways.

Poly(beta-amino esters) (PBAE) polymers are widely used in biomedical fields due to their biocompatibility and biodegradability. For example, U.S. Patent 7,427,394 B2 relates to PBAE prepared from the conjugate addition of bis(secondary amines) or primary amines to a bis(acrylate ester). Methods of preparing these polymers from commercially available starting materials are also provided in this disclosure. The patentees suggest that the PBAE polymers may also be used to encapsulate other agents to be delivered. They are particularly useful in delivering labile agents given their ability to buffer the pH of their surroundings.

U.S. Patent 8,808,681 B2 relates to acrylate-terminated PBAE cross-linked to form materials useful in the medical as well as non-medical field. The resulting materials due to the hydrolysable ester bond in the polymer backbone are biodegradable under physiological conditions. These cross-linked materials are particular useful as drug delivery vehicles, tissue engineering scaffolds, and in fabricating microdevices. The materials may also be used as plastics, coating, adhesives, inks, etc. The cross-linked materials exhibit a wide range of degradation times, mass loss profiles, and mechanical properties. Therefore, the properties of the material may be tuned for the desired use. The high-throughput approach to prepare a library of cross-linked PBAE allows for the rapid screening and design of degradable polymers for a variety of applications.

U.S. Patent Application Publication 2019/0125874 relates to PBAE useful as vehicles for the delivery of therapeutic agents, such as nucleic acids. The disclosed polymers form stable compositions, and are suitable for the delivery of therapeutic agents via nebulization.

It would be desirable to have an encapsulation composition with both enhanced degradability and encapsulation performance at the same time.

SUMMARY OF THE INVENTION

The objective of the present invention is to manufacture a consumer product composition comprising PAC/PBAE delivery particles. The delivery particle can have any of a single shell, double shell, or multiple shell structure with improved degradability, encapsulation performance and customized properties.

Exemplary embodiments of the invention are directed to a polyacrylate (PAC) and poly(beta-amino esters) (PAC/PBAE) delivery particles, including a core material; and a shell having a composition including PAC, PBAE, and hybrid PAC/PBAE.

Exemplary embodiments of the invention are also directed to a PAC/PBAE wherein the shell of the delivery particle is derived from i) 5% to 90% of a preformed PBAE prepolymer, or a reaction product of a first multifunctional acrylate and a multifunctional amine ii) 0.1% to 90% of a multifunctional (meth)acrylate monomer, iii) at least one oil soluble or dispersible thermal free radical initiator, iv) 0% to 90% of a water-soluble or dispersible multifunctional acrylate, and v) 0% to 10% of a monofunctional acidic or/and basic (meth)acrylate monomer, by weight of the microcapsule shell.

Exemplary embodiments of the invention are also directed to a PAC/PBAE delivery particle, wherein the preformed PBAE prepolymer is derived from a first multifunctional acrylate and a multifunctional amine or polyamine, wherein a molar ratio of the first multifunctional acrylate to the multifunctional amine is in a range from about 100:1 to about 1:100, preferably from about 10:1 to about 1:10, more preferably from about 2:1 to about 1:2.

Exemplary embodiments of the invention are also directed to a PAC/PBAE delivery particle, wherein, in the

PAC/PBAE prepolymer, the weight ratio of the PAC to the PBAE prepolymer is from about 1:100 to about 1:1.

In Exemplary embodiments the preformed PBAE prepolymer contains free amino moieties reactive with a multifunctional (meth)acrylate, preferably acrylate, via Aza-Michael Addition reaction. In yet further embodiments, the preformed PBAE prepolymer contains free (meth)acrylate moieties reactive with a multifunctional (meth)acrylate, via free radical polymerization.

Exemplary embodiments of the invention are also directed to a PAC/PBAE delivery particle, wherein the shell wherein the PBAE prepolymer of the shell is derived from a water-soluble or dispersible multifunctional acrylate and a multifunctional amine or polyamine, wherein a molar ratio of the first multifunctional acrylate to the multifunctional amine or polyamine is in a range from about 100:1 to about 1:100, preferably from about 10:1 to about 1:10, more preferably from about 2:1 to about 1:2.

Exemplary embodiments of the invention are also directed to a PAC/PBAE delivery particle, wherein the shell includes PAC, PBAE, and/or hybrid PAC/PBAE and has a single shell structure comprising a contiguous covalently-linked hybrid PAC/PBAE structure.

Exemplary embodiments of the invention are directed to a PAC/PBAE delivery particle, wherein the shell has an inner surface and an outer surface, or a dual shell structure including an inner shell and an outer shell, a composition of the dual shell structure includes PAC and hybrid PAC/PBAE in the inner shell or the inner surface and PBAE in the outer shell or the outer surface, wherein the composition of the outer shell or the outer surface crosslinks or deposits to the composition of the inner shell or the inner surface via covalent bond.

Exemplary embodiments of the invention are directed to a PAC/PBAE delivery particle, wherein the shell has a multi-shell structure, a composition of the multi-shell structure includes PAC in an inner shell, hybrid PAC/PBAE in a transitional shell, and PBAE in an outer shell, the composition of each shell crosslinks or deposits to the composition of an adjacent shell.

Exemplary embodiments of the invention are directed to a PAC/PBAE delivery particle, wherein a median particle size of the PAC/PBAE delivery particle is from about 3 to about 100 $\mu$m.

Exemplary embodiments of the invention are directed to a PAC/PBAE delivery particle, wherein a zeta potential of the PAC/PBAE delivery particle is from about -100 mV to about +200mV at pH 3 and from about -200mV to about +100mV at pH 10.

Exemplary embodiments of the invention are directed to a method of producing a PAC/PBAE delivery particle, including:

providing a first aqueous solution comprising an emulsifier and water;
providing a second aqueous solution comprising a multifunctional amine, a first multifunctional acrylate and water, and mixing the second aqueous solution at a first temperature for a first period of time;
adding the first aqueous solution into the second aqueous solution under mixing to obtain a mixture of the first aqueous solution and the second aqueous solution;
providing a first oil phase comprising the core material, a multifunctional (meth)acrylate, an acidic monomeric (meth)acrylate, and a basic monomeric (meth)acrylate;
providing a second oil phase comprising at least one thermal radical initiator and the core material and subjecting the second oil phase for a period time at elevated temperature to activate the thermal radical initiator;
adding the first oil phase into the second oil phase and mixing for a period of time to obtain a combined oil phase;
adding the combined oil phase into the mixture of the first aqueous solution and the second aqueous solution, applying high shear agitation at a second temperature until a target drop size is reached to obtain an emulsion;
providing a third aqueous solution comprising a second multifunctional acrylate, adding the third aqueous solution into the emulsion under mixing; and
increasing a temperature to a third temperature in a second period of time and holding the temperature at the third temperature for a third period of time under mixing.

Exemplary embodiments of the invention are directed to a method of producing a PAC/PBAE delivery particle, wherein the second aqueous solution does not include a multifunctional acrylate.

Exemplary embodiments of the invention are directed to a method of producing a PAC/PBAE delivery particle, wherein the third aqueous solution is not provided and is not added into the emulsion containing the first aqueous solution, the second aqueous solution and the oil phase.

Exemplary embodiments of the invention are directed to a method of producing a PAC/PBAE delivery particle, wherein the multifunctional amine or polyamine is diethylenetriamine, ethylenediamine, triethylenetetramine, pentaethylenehexamine, polyethylenimine, chitosan, chitin, gelatin, arginine, lysine, ornithine, nisin, histidine, and mixtures thereof, the first and the second multifunctional acrylate is diethylene glycol diacrylate, trifunctional trimethylolpropane triacrylate, ethoxylated trimethylolpropane triacrylate independently or a combination therefore.

Exemplary embodiments of the invention are directed to a method of producing a PAC/PBAE delivery particle,

wherein the first temperature is from about 25 to about 70 °C, the second temperature is from about 25 to about 70 °C, the third temperature is from about 50 to about 95 °C, the first period of time is from about 10 to about 360 minutes, the second period of time is from about 30 to about 120 minutes, and the third period of time is from about 2 to about 24 hours.

Exemplary embodiments of the invention are directed to a method of producing the PAC/PBAE delivery particle, including:

providing a first aqueous solution comprising an emulsifier and water;
providing a second aqueous solution comprising a multifunctional amine, a first multifunctional acrylate and water, and mixing the second aqueous solution at a first temperature for a first period of time;
providing a first oil phase comprising the core material, a multifunctional (meth)acrylate, an acidic monomeric (meth)acrylate, and a basic monomeric (meth)acrylate;
providing a second oil phase comprising at least one thermal radical initiator and the core material and subjecting the second oil phase for a period time at elevated temperature to activate the thermal radical initiator;
adding the first oil phase into the second oil phase and mixing for a period of time to obtain a combined oil phase;
adding the combined oil phase into the first aqueous solution, applying high shear agitation at a second temperature until a target drop size is reached, and then switching to mix to obtain a first emulsion;
adding the second aqueous solution into the first emulsion under mixing to obtain a second emulsion;
providing a third aqueous solution comprising a second multifunctional acrylate, and adding the third aqueous solution into the second emulsion under mixing; and
increasing a temperature to a third temperature in a second period of time and hold the temperature at the third temperature for a third period of time under mixing.

Exemplary embodiments of the invention are directed to a method of producing a PAC/PBAE delivery particle, wherein the second aqueous solution does not include a multifunctional acrylate.

In certain embodiments of making the PAC/PBAE delivery particle, nonlimiting examples of the multifunctional (meth)acrylate can be selected from group consisting of tri-functional (meth)acrylate, tetra-functional (meth)acrylate, penta-functional (meth)acrylate, hexa-functional (meth)acrylate, hepta-functional (meth)acrylate, and mixtures thereof. The multifunctional (meth)acrylate may also comprise a multifunctional aliphatic urethane acrylate.

Non-limiting examples of the water-soluble or dispersible multifunctional acrylate can be selected from diethylene glycol diacrylate, triethylene glycol diacrylate, tetraethylene glycol diacrylate, poly(ethylene glycol) diacrylate, trimethylolpropane triacrylate, ethoxylated trimethylolpropane triacrylate independently or a combination thereof.

In certain embodiments the initiator is a peroxy based initiator or an azo-based initiator selected from the group consisting of 2,2'-azobis (isobutylnitrile), 2,2'-azobis(2,4-dimethylpentanenitrile), 2,2'-azobis(2,4-dimethylvaleronitrile), 2,2'-azobis(2-methylpropanenitrile), 2,2'-azobis(2-methylbutyronitrile), 1,1'-azobis(cyclohexanecarbonitrile), 1,1'-azobis(cyanocyclohexane), 4,4'-azobis(4-cyanovaleric acid) and mixtures thereof.

A method of producing a PAC/PBAE delivery particle is disclosed, comprising:

providing a first aqueous solution comprising an emulsifier and water;
providing a first oil phase comprising the core material, a multifunctional (meth)acrylate, an acidic monomeric (meth)acrylate, and a basic monomeric (meth)acrylate;
providing a second oil phase comprising the core material and at least one oil soluble or dispersible thermal free radical initiator at elevated temperature for a sufficient period of time to form free radicals;
adding the first oil phase into the second oil phase under mixing at elevated temperature for a period of time to obtain a mixture of the first and second oil phase;
providing a third oil phase comprising a preformed PBAE prepolymer that contains free acrylate moieties. The preformed PBAE prepolymer comprising a reaction product by Aza-Michael addition reaction between a multifunctional amine, and a multifunctional acrylate;
adding the third oil phase into the mixture of the first oil phase and the second oil phase and mixing for a period of time;
adding the mixture of the first oil phase, the second oil phase and the third oil phase into the first aqueous solution, applying high shear agitation until a target particle size is reached to obtain an emulsion at a second temperature, the emulsion comprising an interface;
increasing a temperature to a third temperature in a second period of time and holding the temperature at the third temperature for a third period of time under mixing.

In certain embodiments, the multifunctional amine is selected from3-methyl-4-(3-methylphenyl)piperazine, 4-(diphenylmethyl)piperazine, 4-(ethoxycarbonyl)piperazine, 4-(ethoxycarbonylmethyl)piperazine, 4-(phenylmethyl)piperazine, 4-(1-phenylethyl)piperazine, 4-(1,1-dimethylethoxycarbonyl)piperazine, 4-(2-(bis-(2-propenyl)amino)ethyl)piperazine, 4-(2-(diethylamino)ethyl)piperazine, 4-(2-ethoxyphenyl)piperazine, 4-(2-ethylphenyl)piperazine, 4-(2-hydroxyethyl)piperazine, 4-(2-methoxyethyl)piperazine, 4-(2-methoxyphenyl)piperazine, 4-(2-methylphenyl)piperazine, 4-(2-methylthiophenyl)piperazine, 4-(2-phenylethyl)piperazine, 4-(2,3-dimethylphenyl)piperazine, 4-cyclohexylpiperazine, 4-ethylpiperazine, 4-hydroxy-4-phenylpiperidine, 4-hydroxypyrrolidine, 4-methylpiperazine, 4-phenylpiperazine, piperazine, diethylenetriamine, ethylenediamine, tetraethylenepentaamine, pentaethylenehexamine, polyethylenimine, chitosan, chitin, gelatin, arginine, lysine, ornithine, nisin, or histidine, the water-soluble or dispersible multifunctional acrylate is selected from diethylene glycol diacrylate, triethylene glycol diacrylate, tetraethylene glycol diacrylate, poly(ethylene glycol) diacrylate, trimethylolpropane triacrylate, ethoxylated trimethylolpropane triacrylate, or a combination thereof, the multifunctional (meth)acrylate monomer and /or oligomer is selected from group consisting of tri-functional (meth)acrylate, tetra-functional (meth)acrylate, penta-functional (meth)acrylate, hexa-functional (meth)acrylate, hepta-functional (meth)acrylate, and mixtures thereof.

In certain embodiments, the preformed PBAE prepolymer contains free amino moieties that react with the multifunctional (meth)acrylate in the oil phase, or at the interface via Aza-Michael Addition reaction.

The preformed PBAE prepolymer can further comprise free (meth)acrylate moieties that react with the multifunctional (meth)acrylate via free radical polymerization in the oil phase or at the interface. The preformed PBAE prepolymer also contains free amino moieties that react with the multifunctional acrylate in the water phase via Aza-Michael Addition reaction.

Exemplary embodiments of the invention are directed to a method of producing a PAC/PBAE delivery particle, wherein the multifunctional amine or polyamine is diethylenetriamine, ethylenediamine, triethylenetetramine, pentaethylenehexamine, polyethylenimine, chitosan, chitin, gelatin, arginine, lysine, ornithine, nisin, histidine, the first and the second multifunctional acrylate is diethylene glycol diacrylate, trifunctional trimethylolpropane triacrylate, ethoxylated trimethylolpropane triacrylate, or a combination thereof independently.

Exemplary embodiments of the invention are directed to a method of producing a PAC/PBAE delivery particle, wherein the first temperature is from about 25 to about 70 °C, the second temperature is from about 25 to about 70 °C, the third temperature is from about 50 to about 95 °C, the first period of time is from about 10 to about 360 minutes, the second period of time is from about 30 to about 120 minutes, and the third period of time is from about 2 to about 24 hours.

Exemplary embodiments of the invention are directed to an article of manufacture incorporating the PAC/PBAE delivery particles.

Exemplary embodiments of the invention are directed to the article of manufacture incorporating the PAC/PBAE delivery particles, wherein the article is a soap, a surface cleaner, a laundry detergent, a fabric softener, a shampoo, a textile, a paper towel, an adhesive, a wipe, a diaper, a feminine hygiene product, a facial tissue, a pharmaceutical, a napkin, a deodorant, a heat sink, a foam, a pillow, a mattress, bedding, a cushion, a cosmetic, a medical device, packaging, an agricultural product, a cooling fluid, a wallboard, or an insulation.

The above as well as additional objectives, features, and advantages of the present invention are detailed in the description below.

## DETAILED DESCRIPTION OF THE INVENTION

The present disclosure relates to consumer product compositions comprising populations of delivery particles. The delivery particles (or simply "particles" or "microcapsules", as used herein) are core/shell particles that include a core comprising a benefit agent, and typically a partitioning modifier, and a polymer wall encapsulating said core.

The present invention is based on the discovery that encapsulation using PBAE prepolymer, produced by a polymerization reaction of multifunctional amines with multifunctional acrylates, further polymerizing with multifunctional acrylates and methacrylates by Aza-Michael addition and radical polymerization can yield delivery particles with improved encapsulation performance and enhanced degradability.

### Definitions

As used herein, the articles "a" and "an" when used in a claim, are understood to mean one or more of what is claimed or described. As used herein, the terms "include," "includes," and "including" are meant to be non-limiting. The compositions of the present disclosure can comprise, consist essentially of, or consist of, the components of the present disclosure.

The terms "substantially free of' or "substantially free from" may be used herein. This means that the indicated material is at the very minimum not deliberately added to the composition to form part of it, or, preferably, is not present at analytically detectable levels. It is meant to include compositions whereby the indicated material is present only as an impurity in one of the other materials deliberately included. The indicated material may be present, if at all, at a level of less than 1%, or less than 0.1%, or less than 0.01%, or even 0%, by weight of the composition.

As used herein, "consumer product," means baby care, beauty care, fabric & home care, family care, feminine care, and/or health care products or devices intended to be used or consumed in the form in which it is sold, and not

intended for subsequent commercial manufacture or modification. Such products include but are not limited to diapers, bibs, wipes; products for and/or methods relating to treating human hair, including bleaching, coloring, dyeing, conditioning, shampooing, styling; deodorants and antiperspirants; personal cleansing; skin care including application of creams, lotions, and other topically applied products for consumer use; and shaving products, products for and/or methods relating to treating fabrics, hard surfaces and any other surfaces in the area of fabric and home care, including: air care, car care, dishwashing, fabric conditioning (including softening), laundry detergency, laundry and rinse additive and/or care, hard surface cleaning and/or treatment, and other cleaning for consumer or institutional use; products and/or methods relating to bath tissue, facial tissue, paper handkerchiefs, and/or paper towels; tampons, feminine napkins; adult incontinence products; products and/or methods relating to oral care including toothpastes, tooth gels, tooth rinses, denture adhesives, tooth whitening; over-the-counter health care including cough and cold remedies; pest control products; and water purification.

As used herein, "other than a consumer product" means feedstocks used neat or used for manufacture of industrial or agricultural products. Such feedstocks include dry delivery particles, delivery particles, slurries of delivery particles, delivery particle aggregates, delivery particle powders, delivery particle dispersions, delivery particle coating and binding materials with delivery particles. End use applications can include, but are not limited to, coatings for substrates, raw material slurries, slurries of benefit agent delivery parties for benefit agents such as industrial lubricants such as for injection wells, cakes or powders of benefit agent delivery particles as raw materials in the manufacture of consumer or other products, slurries for delivery of beneficial agents such as slurries for industrial uses such as delivery of fragrances, agricultural actives, lubricants or other actives.

As used herein, the term "cleaning composition" includes, unless otherwise indicated, granular or powder-form all-purpose or "heavy-duty" washing agents, especially cleaning detergents; liquid, gel or paste-form all-purpose washing agents, especially the so-called heavy-duty liquid types; liquid fine-fabric detergents; hand dishwashing agents or light duty dishwashing agents, especially those of the high-foaming type; machine dishwashing agents, including the various pouches, tablet, granular, liquid and rinse-aid types for household and institutional use; liquid cleaning and disinfecting agents, including antibacterial hand-wash types, cleaning bars, mouthwashes, denture cleaners, dentifrice, car or carpet shampoos, bathroom cleaners; hair shampoos and hair-rinses; shower gels and foam baths and metal cleaners; as well as cleaning auxiliaries such as bleach additives and "stain-stick" or pre-treat types, substrate-laden products such as dryer added sheets, dry and wetted wipes and pads, nonwoven substrates, and sponges; as well as sprays and mists.

As used herein, the term "fabric care composition" includes, unless otherwise indicated, fabric softening compositions, fabric enhancing compositions, fabric freshening compositions and combinations thereof. The form of such compositions includes liquids, gels, beads, powders, flakes, and granules.

As used herein, the phrase "benefit agent containing delivery particle" encompasses microcapsules having a core comprising a benefit agent, for example perfume including but not limited to microcapsules encapsulating perfumes, lubricants, oils, waxes, hydrocarbons, essential oils, lipids, skin coolants, sunscreens, antioxidants, malodor reducing agents, odor-controlling materials, fragrances, insect and moth repelling or controlling agents, agricultural actives, colorants, bodying agents, wrinkle control agents, sanitization agents, disinfecting agents, germ control agents, mold control agents, mildew control agents, antiviral agents, drying agents, stain resistance agents, soil release agents, fabric refreshing agents and freshness extending agents, chlorine bleach odor control agents, dye fixatives, dye transfer inhibitors, optical brighteners, color restoration/rejuvenation agents, anti-fading agents, whiteness enhancers, anti-abrasion agents, wear resistance agents, UV protection agents, sun fade inhibitors, enzymes, water proofing agents, shrinkage resistance agents, antibacterial actives, antiperspirant actives, dyes, and mixtures thereof.

As used herein, the terms "particle", "delivery particle" "benefit agent containing delivery particle", "encapsulate", "capsule" and "microcapsule" are synonymous, unless indicated otherwise.

As used herein, reference to the term "(meth)acrylate" or "(meth)acrylic" is to be understood as referring to both the acrylate and the methacrylate versions of the specified monomer, oligomer and/or prepolymer. For example, "allyl (meth)acrylate" indicates that both allyl methacrylate and allyl acrylate are possible, similarly reference to alkyl esters of (meth)acrylic acid indicates that both alkyl esters of acrylic acid and alkyl esters of methacrylic acid are possible, similarly poly(meth)acrylate indicates that both polyacrylate and polymethacrylate are possible. Poly(meth)acrylate materials are intended to encompass a broad spectrum of polymeric materials including, for example, polyester poly(meth)acrylates, urethane and polyurethane poly(meth)acrylates methylcyanoacrylate, ethylcyanoacrylate, diethyleneglycol di(meth)acrylate, ethylene glycol di(meth)acrylate, allyl (meth)acrylate, glycidyl (meth)acrylate, (meth)acrylate functional silicones, di-, tri- and tetraethylene glycol di(meth)acrylate, dipropylene glycol di(meth)acrylate, polyethylene glycol di(meth)acrylate, di(pentamethylene glycol) di(meth)acrylate, ethylene di(meth)acrylate, neopentyl glycol di(meth)acrylate, trimethylol propane tri(meth)acrylate, ethoxylated bisphenol A di(meth)acrylates, bisphenol A di(meth)acrylates, diglycerol di(meth)acrylate, tetraethylene glycol dichloroacrylate, 1,3-butanediol di(meth)acrylate, neopentyl di(meth)acrylate, and various multifunctional(meth)acrylates. Monofunctional (meth)acrylates, i.e., those containing only one (meth)acrylate group, may also be advantageously used. Typical mono(meth)acrylates include 2-ethylhexyl (meth)acrylate, 2-hydroxyethyl (meth)acrylate, cyanoethyl (meth)acrylate, 2-hydroxypropyl (meth)acrylate, p-dimethylaminoethyl (meth)acrylate, lauryl (meth)acrylate, cyclohexyl (meth)acrylate, tetrahydrofurfuryl (meth)acrylate, chlorobenzyl (meth)acrylate, aminoalkyl(meth)acrylate, various alkyl(meth)acrylates and glycidyl (meth)acrylate. Mixtures of (meth)acrylates or their derivatives as well as combinations of one or more (meth)acrylate monomers, oligomers and/or prepolymers or their derivatives with other copolymerizable monomers, including acrylonitriles and methacrylonitriles may be used as well.

For ease of reference in this specification and in the claims, the term "monomer" or "monomers" as used herein with regard to the polymer wall is to be understood as monomers but also is inclusive of oligomers or monomers, and prepolymers formed of the specific monomers.

All temperatures herein are in degrees Celsius (°C) unless otherwise indicated. Unless otherwise specified, all measurements herein are conducted at 20 °C and under atmospheric pressure.

In all embodiments of the present disclosure, all percentages are by weight of the total composition, unless specifically stated otherwise. All ratios are weight ratios, unless specifically stated otherwise.

As used herein the term "water-soluble material" means a material that has a solubility of at least 0.5% wt in water at 60 °C.

As used herein the term "oil soluble" means a material that has a solubility of at least 0.1% wt in the core of interest at 50 °C.

As used herein the term "oil dispersible" means a material that can be dispersed at least 0.1% wt in the core of interest at 50 °C without visible agglomerates.

As used herein, the articles including "a" and "an" when used in a claim, are understood to mean one or more of what is claimed or described.

As used herein, the terms "include", "includes" and "including" are meant to be non-limiting.

The test methods disclosed in the Test Methods section of the present application should be used to determine the respective values of the parameters of the invention.

Unless otherwise noted, all component or composition levels are in reference to the component or composition exclusive of impurities, for example, residual solvents or by-products, which may be present in commercially available sources of such components or compositions.

As used herein "biodegradable" refers to a material that has above 30% CO2 release according to the OECD301B test method.

It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

Multifunctional amines herein are to be understood including any monomer, oligomer, or polyamine that has one or multiple primary amines groups and one or multiple secondary amines groups. Multifunctional acrylate is to be understood as referring to a compound having at least two acrylate groups and comprising multifunctional acrylate monomer, oligomer and/or prepolymer.

## CONSUMER PRODUCT COMPOSITION

The present disclosure relates to a consumer product composition that comprises a population of delivery particles and a treatment adjunct as described in more detail below.

### Delivery particle

Specifically, in the invention, a multifunctional amine polymerizes with a multifunctional acrylate, under heating, to form a PBAE prepolymer. In addition, a multifunctional (meth)acrylate self-polymerizes via free radical polymerization to form a PAC delivery particle shell or with the primary or secondary amine moiety of PBAE prepolymer to form PBAE or hybrid PAC/PBAE in or on the delivery particle shell. Further, the acrylate moiety of the PBAE and excess multifunctional acrylate if any also compete with multifunctional (meth)acrylate in reaction with the multifunctional amine and the primary and the secondary amine moiety of PBAE. These competing reactions can be advantageously employed to create a PAC/PBAE delivery particle including PAC, hybrid PAC/PBAE and PBAE with a single shell, advantageously dual shells, or multiple shells through the control of the relative amounts of different reactants, the presence or absence of the prepolymerization step, and the reaction sequence in forming the delivery particles. A unique delivery particle with customized properties is thereby achievable.

In one embodiment, the PAC/PBAE delivery particle shell contains i) 5% to 90% of a preformed PBAE prepolymer, or a reaction product of a first multifunctional acrylate and a multifunctional amine, or a polyamine ii) 0.1% to 90%

of a multifunctional (meth)acrylate monomer, iii) 0.001% to 5% one oil soluble or dispersible thermal free radical initiator, iv) 0.1% to 90% of a water-soluble or dispersible multifunctional acrylate, and v) 0% to 10% of a monofunctional acidic or/and basic (meth)acrylate monomer, by weight of the delivery particle shell, provided that the sum is 100%.

In a first method of preparing delivery particles according to the invention, an oil soluble PBAE prepolymer or polymer is prepared by reacting a bifunctional secondary amine, e.g. piperazine, or a monofunctional primary amine, e.g. ethylamine, with a bifunctional acrylate, for example diethylene glycol diacrylate, in oil at elevated temperature (35 °C) for a period of time, such as 2 hours. The molar ratio of acrylate group to secondary and primary amine is maintained as slightly greater than 1 to ensure the end of PBAE polymer is capped with acrylate groups. The oil soluble PBAE polymer can also be prepared by reacting multifunctional amine like diethylenetriamine, ethylenediamine, triethylenetetramine, aminoethylpiperazine, N,N'-Bis-(2-aminoethyl)piperazine), tris(2-aminoethyl)amine, or polyethylenimine with bifunctional acrylate or other multifunctional acrylate. This oil soluble PBAE polymer can be linear or branched depending on the selection of amine and acrylate.

The preformed oil soluble PBAE polymer is then reacted with multifunctional (meth)acrylate in oil phase via free radical polymerization between the acrylate moieties on the PBAE polymer and multifunctional acrylate or Aza-Michael addition between the remaining amine moieties and the acrylate moieties of the multifunctional acrylate. The remaining acrylate moieties on the multifunctional acrylate can be further radical polymerized to form polyacrylate shell.

An oil in water emulsion is created by adding the oil phase or combined oil phase into a water phase that contains an emulsifier. The emulsion is then mixed under high shear to reach the target particle size and then thermally cured to form capsule.

In the second method of preparing delivery particles according to the invention, a water-soluble PBAE prepolymer is first prepared by reacting multifunctional amine, such as diethylenetriamine, chitosan, chitin, or gelatin with bifunctional acrylate, such as diethylene glycol diacrylate in water at elevated temperature for certain time. The molar amount of the acrylate can be equal or slightly greater than the molar amount of primary amine in the multifunctional amine.

A water phase was created by mixing an emulsifier, such as polyvinyl alcohol (PVA) and the preformed PBAE polymer solution in water.

A first oil phase as prepared by mixing an active ingredient, namely a benefit agent such as perfume, herbicide, pesticide or other active, with a multifunctional acrylate at room temperature. A second oil phase is created by mixing a free radical initiator, such as a Vazo initiator, in oil and optionally a diluent at elevated temperature to activate the free radical initiator. The two oil phases are then combined for a short period of time to form an acrylate or methacrylate prepolymer.

The water phase that contains the PBAE prepolymer, and an emulsifier is then added to the oil phase and mixed for a sufficient period of time under high shear to enable the reaction between the free amine moieties on the PBAE prepolymer and the acrylate moieties on the multifunctional (meth)acrylate or (meth)acrylate prepolymer at the oil/water interface.

Optionally, a third mono, bi or multifunctional water-soluble or dispersible acrylate can be added to the emulsion to react with any remaining amine moieties on the PBAE prepolymer to further polymerize or crosslink the PBAE prepolymer.

The emulsion is then heated to elevated temperature for a period of time to cure the delivery particle shell by forming a polyacrylate shell from the oil phase and PBAE shell from the water and interphase. The PBAE shell forming materials are covalently bonded.

The PAC/PBAE delivery particle encapsulates a core. To encapsulate the core, a first aqueous solution is prepared by mixing an emulsifier with water. During the formation of the polymer wall of the PAC/PBAE delivery particles, the emulsifier can become entrapped in the polymer wall material. These inclusions of emulsifier into the polymer wall usefully can be used to advantage in modification of polymer wall properties, influencing such attributes as flexibility, leakage, strength, and other properties. Thus, the polymer wall of the PAC/PBAE delivery particles may further comprise an emulsifier entrapped in the polymer wall, preferably wherein the emulsifier comprises polyvinyl alcohol.

Non-limiting examples of emulsifiers include water-soluble salts of alkyl sulfates, alkyl ether sulfates, alkyl isothionates, alkyl carboxylates, alkyl sulfosuccinates, alkyl succinamates, alkyl sulfate salts such as sodium dodecyl sulfate, alkyl sarcosinates, alkyl derivatives of protein hydrolyzates, acyl aspartates, alkyl or alkyl ether or alkylaryl ether phosphate esters, sodium dodecyl sulphate, phospholipids, lecithin, soaps, sodium, potassium or ammonium stearate, oleate, palmitate, alkylarylsulfonic acid salts such as sodium dodecylbenzenesulfonate, sodium dialkylsulfosuccinates, dioctyl sulfosuccinate, sodium dilaurylsulfosuccinate, poly(styrene sulfonate) sodium salt, isobutylene-maleic anhydride copolymer, gum arabic, sodium alginate, carboxymethylcellulose, cellulose sulfate and pectin, poly(styrene sulfonate), isobutylene-maleic anhydride copolymer, carrageenan, sodium alginate, pectic acid, tragacanth gum, almond gum and agar; semi-synthetic polymers such as carboxymethyl cellulose, sulfated cellulose, sulfated methylcellulose, carboxymethyl starch, phosphated starch, lignin sulfonic acid; synthetic polymers such as maleic anhydride copolymers (including hydrolyzates thereof), polyacrylic acid, polymethacrylic acid, acrylic acid butyl acrylate copolymer or crotonic acid homopolymers and copolymers, vinyl benzenesulfonic acid or 2-acrylamido-2-methylpropanesulfonic acid homopolymers and copolymers, and partial amide or partial ester of such polymers and copolymers, carboxy modified polyvinyl alcohol, sulfonic acid-modified polyvinyl alcohol and phosphoric acid-modified polyvinyl alcohol, phosphated or sulfated tristyrylphenol ethoxylates, palmitamidopropyltrimonium chloride (Varisoft PATC™, available from Degussa Evonik, Essen, Germany), distearyl dimonium chloride, cetyltrimethylammonium chloride, quaternary ammonium compounds, fatty amines, aliphatic ammonium halides, alkyldimethylbenzylammonium halides, alkyldimethylethylammonium halides, polyethylenimine, poly(2-dimethylamino)ethyl methacrylate) methyl chloride quaternary salt, poly(1-vinylpyrrolidone-co-2-dimethylaminoethyl methacrylate), poly(acrylamide-co-diallyldimethylammonium chloride), poly(allylamine), poly[bis(2-chloroethyl) ether-alt-1,3-bis[3-(dimethylamino)propyl]urea] quaternized, and poly(dimethylamine-co-epichlorohydrin-co-ethylenediamine), condensation products of aliphatic amines with alkylene oxide, quaternary ammonium compounds with a long-chain aliphatic radical, e.g. distearyldiammonium chloride, and fatty amines, alkyldimethylbenzylammonium halides, alkyldimethylethylammonium halides, polyalkylene glycol ether, condensation products of alkyl phenols, aliphatic alcohols, fatty acids with alkylene oxide, ethoxylated alkyl phenols, ethoxylated aryl phenols, ethoxylated polyaryl phenols, carboxylic esters solubilized with a polyol, polyvinyl alcohol, polyvinyl acetate, or copolymers of polyvinyl alcohol polyvinyl acetate, polyacrylamide, poly(N-isopropylacrylamide), poly(2-hydroxypropyl methacrylate), poly(-ethyl-2-oxazoline), poly(2-isopropenyl-2-oxazoline-co-methyl methacrylate), poly(methyl vinyl ether), poly(vinyl alcohol-co-ethylene), or cocoamidopropyl betaine. Especially useful polyvinyl alcohols include polyvinyl alcohols of molecular 13,000 to 1,876,000 Daltons, preferably from 13,000 to about 230,000 Daltons, or even from 146,000 to 186,000 Daltons. The polyvinyl alcohol can be partially or fully hydrolyzed. Polyvinyl alcohol partially hydrolyzed in the range of 80 to 95% hydrolyzed is preferred, even more preferred 87% to 89% hydrolyzed.

Emulsifier, if employed, is typically from about 0.1 to about 40% by weight, preferably from about 0.2 to about 15% by weight, more typically from about 0.5 to about 10% by weight, based on the total weight of the delivery particle slurry.

In one embodiment of the present invention, the emulsifier is polyvinyl alcohol such as Selvol™ Polyvinyl Alcohol 540 (Sekisui Specialty Chemicals America, LLC).

A second aqueous solution containing water-soluble PBAE prepolymer is prepared by dissolving a multifunctional amine in water under mixing at a temperature of from about 25°C to about 70 °C. Then, a multifunctional acrylate, for example, a bifunctional acrylate, is added into the multifunctional amine solution. A prepolymer is formed by mixing the multifunctional amine and the multifunctional acrylate for a period of time of from about 10 to about 360 mins at a temperature of from about 25 to about 70 °C. The molar ratio of the multifunctional acrylate to the multifunctional amine is from about 100:1 to about 1:100, preferably from about 10:1 to about 1:10, more preferably from about 2:1 to about 1:2.

During the above described step, a water-soluble or dispersible PBAE prepolymer is produced by the polymerization reaction between the amine group of the multifunctional amine monomer, oligomer, prepolymer or polymer and the acrylate group of a multifunctional acrylate. Generally, the reaction here proceeds by aza-Michael addition to the $\beta$-carbon atom of $\alpha,\beta$-unsaturated carbonyls of the multifunctional acrylate.

As used herein, reference to the term "PAC" or "acrylate" or "acrylic" is to be understood as referring to the acrylate version of the specified monomer, oligomer, polymer and/or prepolymer. For example, multifunctional acrylate, alkyl esters of acrylic acid, and polyacrylate. Each alkyl moiety herein, unless otherwise indicated, can be from $C_1$ to $C_8$, or even from $C_1$ to $C_{24}$. Polyacrylate materials are intended to encompass a broad spectrum of polymeric materials including, for example, polyester polyacrylates, methyl cyanoacrylate, ethyl cyanoacrylate, diethylene glycol diacrylate, ethylene glycol diacrylate, allyl acrylate, glycidyl acrylate, acrylate functional silicones, di-, tri- and tetra ethylene glycol diacrylate, dipropylene glycol diacrylate, polyethylene glycol diacrylate, di(penta methylene glycol) diacrylate, ethylene diacrylate, neopentyl glycol diacrylate, trimethylolpropane triacrylate, ethoxylated bisphenol A diacrylates, bisphenol A diacrylates, diglycerol diacrylate, tetra ethylene glycol dichloroacrylate, 1,3-butanediol diacrylate, neopentyl diacrylate, trimethylolpropane triacrylate, polyethylene glycol diacrylate, dipropylene glycol diacrylate, various multifunctional acrylates, and multifunctional amine acrylates.

Non-limiting examples of multifunctional acrylate monomers, oligomers and prepolymers thereof include ethylene glycol diacrylate, trimethylolpropane triacrylate, trimethylolpropane triacrylate, pentaerythritol tetraacrylate, tricyclodecane dimethanol diacrylate, 1,10 decanediol diacrylate, 1,6 hexanediol diacrylate, 1,9 nonanediol diacrylate, neopentyl glycol diacrylate, di-trimethylolpropane tetraacrylate, dipentaerythritol pentaacrylate, ethoxylated (2) bisphenol A diacrylate, 2,2 bis[4-(acyloyl ethoxy) phenyl] propane, ethoxylated (3) bisphenol A diacrylate, dipropylene glycol diacrylate, ethoxylated (4) bisphenol A diacrylate, ethoxylated (4) bisphenol A diacrylate, 2,2 bis[4-(acyloyl ethoxy) phenyl] propane, pentaerythritol triacrylate, polyethylene glycol 200 diacrylate, ethoxylated (9) trimethylolpropane triacrylate, 2,2 bis[4-(acyloyl ethoxy) phenyl] propane, ethoxylated (30) BPA diacrylate, ethoxylated (15) trimethylolpropane triacrylate, ethoxylated glycerine triacrylate, ethoxylated (20) trimethylolpropane triacrylate, polyethylene glycol 400 diacrylate, polyethylene glycol 600 diacrylate, ethoxylated glycerine triacrylate, ethoxylated pentaerythritol tetraacrylate, polyethylene glycol 1000 diacrylate, polyethylene (200) glycol diacrylate, polyethylene glycol (200) diacrylate, polyethylene glycol (400) diacrylate, polyethylene glycol (600) diacrylate and tris (2-hydroxy ethyl) isocyanurate triacrylate, diethylene glycol diacrylate, ethoxylated (3) trimethylolpropane triacrylate, polypropylene glycol 400 diacrylate, ethoxylated (10)

bisphenol A diacrylate, ethoxylated (10) bisphenol A diacrylate, 2,2 bis[4-(acryloyl ethoxy) phenyl] propane, ethoxylated (4) pentaerythritol tetraacrylate, triethylene glycol diacrylate, 2-hydroxyl 1-3 diacryloxy propane, ethoxylated (6) trimethylolpropane triacrylate, ethoxylated propyleneglycol diacrylate, 2,2 bis[4-(acryloyl ethoxy) phenyl] propane and the like, and mixtures of any of the foregoing.

As used herein, reference to the term "PAC" is to be understood as referring to the acrylate version of the specified monomer, oligomer, polymer and/or prepolymer. The skilled artisan will appreciate that the PAC monomers, oligomers, polymers and/or prepolymers may be further free radically polymerized with various (meth)acrylates herein described, and such variations are intended by the term as used herein.

As used herein, reference to the term "PBAE" is to be understood as referring to the monomer, oligomer, prepolymer and/or polymer versions of beta-amino esters.

Non-limiting examples of multifunctional amine or polyamine is diethylenetriamine, ethylenediamine, triethylenetetramine, tetraethylenepentamine, pentaethylenehexamine, polyethylenimine, chitosan, chitin, gelatin, arginine, lysine, ornithine, nisin, histidine, or similar amines that have one or multiple primary amines and/or one or multiple secondary amines.

In one embodiment, the molar amount of the acrylate group of the multifunctional acrylate is the same as the molar amount of the primary amine of the multifunctional amine.

The delivery particles of the present disclosure include a core. The core may comprise a benefit agent. Suitable benefit agents located in the core may include benefit agents that provide benefits to a surface, such as a fabric or hair.

The core may comprise from about 40% to about 100%, preferably from about 50% to about 95%, more preferably from about 50% to about 80%, by weight of the core, of the benefit agent.

The benefit agent may be selected from the group consisting of fragrance, silicone oils, waxes, hydrocarbons, higher fatty acids, essential oils, lubricants, lipids, skin coolants, vitamins, sunscreens, antioxidants, glycerine, catalysts, bleach particles, silicon dioxide particles, malodor reducing agents, odor-controlling materials, chelating agents, antistatic agents, softening agents, insect and moth repelling agents, colorants, antioxidants, chelants, bodying agents, drape and form control agents, smoothness agents, wrinkle control agents, sanitization agents, disinfecting agents, germ control agents, mold control agents, mildew control agents, antiviral agents, drying agents, stain resistance agents, soil release agents, fabric refreshing agents and freshness extending agents, chlorine bleach odor control agents, dye fixatives, dye transfer inhibitors, color maintenance agents, optical brighteners, color restoration/rejuvenation agents, anti-fading agents, whiteness enhancers, anti-abrasion agents, wear resistance agents, fabric integrity agents, anti-wear agents, anti-pilling agents, defoamers, anti-foaming agents, UV protection agents, sun fade inhibitors, anti-allergenic agents, enzymes, water proofing agents, fabric comfort agents, shrinkage resistance agents, stretch resistance agents, stretch recovery agents, skin care agents, glycerin, synthetic or natural actives, antibacterial actives, antiperspirant actives, cationic polymers, dyes, and mixtures thereof. Preferably the benefit agent comprises fragrance, essential oils and mixtures thereof.

The encapsulated benefit agent may preferably a fragrance, which may include one or more perfume raw materials. The term "perfume raw material" (or "PRM") as used herein refers to compounds having a molecular weight of at least about 100 g/mol and which are useful in imparting an odor, fragrance, essence or scent, either alone or with other perfume raw materials. Typical PRMs comprise inter alia alcohols, ketones, aldehydes, esters, ethers, nitriles and alkenes, such as terpene. A listing of common PRMs can be found in various reference sources, for example, "Perfume and Flavor Chemicals", Vols. I and II; Steffen Arctander Allured Pub. Co. (1994) and "Perfumes: Art, Science and Technology", Miller, P. M. and Lamparsky, D., Blackie Academic and Professional (1994).

The PRMs may be characterized by their boiling points (B.P.) measured at the normal pressure (760 mm Hg), and their octanol/water partitioning coefficient (P), which may be described in terms of logP, determined according to the test method below. Based on these characteristics, the PRMs may be categorized as Quadrant I, Quadrant II, Quadrant III, or Quadrant IV perfumes, as described in more detail below.

The fragrance may comprise perfume raw materials that have a logP of from about 2.5 to about 4. It is understood that other perfume raw materials may also be present in the fragrance.

The perfume raw materials may comprise a perfume raw material selected from the group consisting of perfume raw materials having a boiling point (B.P.) lower than about 250 °C and a logP lower than about 3, perfume raw materials having a B.P. of greater than about 250 °C and a logP of greater than about 3, perfume raw materials having a B.P. of greater than about 250 °C and a logP lower than about 3, perfume raw materials having a B.P. lower than about 250 °C and a logP greater than about 3, and mixtures thereof. Perfume raw materials having a boiling point B.P. lower than about 250 °C and a logP lower than about 3 are known as Quadrant I perfume raw materials. Quadrant 1 perfume raw materials are preferably limited to less than 30% of the perfume composition. Perfume raw materials having a B.P. of greater than about 250 °C and a logP of greater than about 3 are known as Quadrant IV perfume raw materials, perfume raw materials having a B.P. of greater than about 250°C and a logP lower than about 3 are known as Quadrant II perfume raw materials, perfume raw materials having a B.P. lower than about 250 °C and a logP greater than about 3 are known as a Quadrant III perfume raw materials. Suitable Quadrant I, II, III and IV perfume raw materials are disclosed in U.S.

Patent 6,869,923 B1.

The core of the delivery particles of the present disclosure may further comprise a partitioning modifier. The properties of the partitioning modifier in the core can play a role in determining how much, how quickly, and/or how permeable the PAC, PBAE, PAC/PBAE shell material will be when established at the oil/water interface. For example, if the oil phase comprises highly polar materials, these materials may reduce the diffusion of the acrylate oligomers and polymers to the oil/water interface and result in a very thin, highly permeable shell. Incorporation of a partitioning modifier can adjust the polarity of the core, thereby changing the partition coefficient of the polar materials in the partitioning modifier versus the acrylate oligomers, and can result in the establishment of a well-defined, highly impermeable shell. The partitioning modifier may be combined with the core's benefit agent prior to incorporation of the wall-forming monomers.

The partitioning modifier may be present in the core at a level of from about 5% to about 60%, preferably from about 20% to about 50%, more preferably from about 30% to about 50%, by weight of the core.

The partitioning modifier may comprise a material selected from the group consisting of vegetable oil, modified vegetable oil, mono-, di-, and tri-esters of C4-C24 fatty acids, isopropyl myristate, dodecanophenone, lauryl laurate, methyl behenate, methyl laurate, methyl palmitate, methyl stearate, and mixtures thereof. The partitioning modifier may preferably comprise or even consist of isopropyl myristate. The modified vegetable oil may be esterified and/or brominated. The modified vegetable oil may preferably comprise castor oil and/or soybean oil. US Patent Application Publication 20110268802, incorporated herein by reference, describes other partitioning modifiers that may be useful in the presently described delivery particles. An emulsion containing the first aqueous solution, the second aqueous solution and the oil phase can be prepared by different processes.

One process to produce the emulsion is first combining the first aqueous solution containing the emulsifier and the second aqueous solution containing the PBAE prepolymer under mixing, and then adding the combined oil phase containing the active material, the oil soluble thermal initiator, the acidic and/or basic (meth)acrylate, and the multifunctional (meth)acrylate into the aqueous mixture of the first aqueous solution and the second aqueous solution. The resultant mixture is milled under high shear agitation at a speed from about 2000 to 4000 rpm at a temperature of 25-70°C. The milling under high shear agitation may take 6 to 61 mins or even longer until a target particle size is reached, and the emulsion is stable. Milling is then stopped and mixing continued. Mixing may continue up to several hours.

Alternatively, another process to produce the emulsion is first mixing the first aqueous solution containing the emulsifier and the combined oil phase containing the active material, the oil soluble thermal initiator, the acidic and/or basic (meth)acrylate, and the multifunctional (meth)acrylate. Milling the resultant mixture under high shear agitation at a speed typically from 2000 to 4000 rpm at a temperature of 25-70°C. The milling may take 6 to 61 mins or even longer until a target particle size is reached, and the emulsion is stable. Milling under high shear agitation is then stopped. Next, the second aqueous solution containing the PBAE prepolymer is mixed into the emulsion containing the first aqueous solution and the oil phase.

In some embodiments, the milling speed under high shear agitation is 2000 rpm, 2500 rpm, 3000 rpm, or 4000 rpm. In some embodiments, the milling temperature is 25°C, 35°C, 50°C or 70C. In some embodiments, the milling takes 10 mins, 30 mins, 60 mins or longer.

Meanwhile, a third aqueous solution is prepared by dissolving a multifunctional acrylate or a mixture of monofunctional and multifunctional acrylates or a mixture of multifunctional acrylate, such as a bifunctional acrylate, or/and a trifunctional acrylate, or/and a monofunctional acrylate in water.

In forming the PAC/PBAE delivery particles of the invention, the basic (meth)acrylate monomer can be selected, by way of illustration and not limitation, from the group consisting of ethylaminoethyl acrylate, ethylaminoethyl methacrylate, aminoethyl acrylate, aminoethyl methacrylate, tertiarybutyl aminoethyl acrylate, tertiarybutyl aminoethyl methacrylate, diethylamino acrylate, diethylamino methacrylate, diethylaminoethyl acrylate diethylaminoethyl methacrylate, dimethylaminoethyl acrylate and dimethylaminoethyl methacrylate, and the acidic (meth)acrylate monomer is selected from the group consisting of 2-carboxyethyl acrylate, 2-carboxyethyl methacrylate, 2-carboxypropyl acrylate, 2-carboxypropyl methacrylate, carboxyoctyl acrylate, carboxyoctyl methacrylate, 2-acryloyloxybenzoic acid, 3-acryloyloxybenzoic acid, 4-acryloyloxybenzoic acid, 2-methacryloyloxybenzoic acid, 3-methacryloyloxybenzoic acid, and 4-methacryloyloxybenzoic acid, 4-acryloyloxyphenylacetic acid, and 4-methacryloyloxyphenylacetic acid.

The acidic (meth)acrylate may comprise, by way of illustration, one or more of carboxy substituted acrylates or methacrylates, preferably carboxy substituted alkyl acrylates or methacrylates, such as carboxyalkyl acrylate, carboxyalkyl methacrylate, carboxyaryl acrylate, carboxy aryl methacrylate, and preferably the alky moieties are straight chain or branched C1 to C10. The carboxyl moiety can be bonded to any carbon of the C1 to C10 alkyl moiety, preferably a terminal carbon. Carboxy substituted aryl acrylates or methacrylates can also be used, or even (meth)acryloyloxyphenylalkylcarboxy acids. The alkyl moieties of the (meth)acryloyloxyphenylalkylcarboxy acids can be C1 to C10.

Suitable carboxy (meth)acrylates for use in particles of the present disclosure may include 2-carboxyethyl acrylate, 2-carboxyethyl methacrylate, 2-carboxypropyl acrylate, 2-carboxypropyl methacrylate, carboxyoctyl acrylate, carboxyoctyl methacrylate. Carboxy substituted aryl acrylates or methacrylates may include 2-acryloyloxybenzoic acid,

3-acryloyloxybenzoic acid, 4-acryloyloxybenzoic acid, 2-methacryloyloxybenzoic acid, 3-methacryloyloxybenzoic acid, and 4-methacryloyloxybenzoic acid. (Meth)acryloyloxyphenylalkylcarboxy acids by way of illustration and not limitation can include 4-acryloyloxyphenylacetic acid or 4-methacryloyloxyphenylacetic acid.

Optionally, but preferably, included in the water and/or oil phases is one or more of an acidic (meth)acrylate and/or an basic (meth)acrylate at a minor concentration of about less than about 5% by weight or even less than about 1% by weight, optimally at a range of from about .001% to about 5% by weight based on the weight of the polymer wall. Minor amounts of other monofunctional (meth)acrylates can also be included to adjust wall properties in specific applications.

Basic (meth)acrylate monomer and acid (meth)acrylate monomer typically are used in a molar proportion from about 3:1 to about 1:3 and together have a percent by weight as compared to the weight of the polymer shell of from 0 to 5% wt based on total polymer shell.

In the process of making the benefit agent delivery particles, assuming a slurry system of about 800 g including an oil phase and/or benefit agent being an oil, the largest constituents are typically the oil(s), with 10 to 70 weight percent, preferably 25 to 55 weight percent the benefit agent; 10 to 70 weight percent, preferably 35 to 65 weight percent water; preferably 0.1 to 10 weight percent, usually 0.5 to 8 weight percent multi-functional (meth)acrylate monomer; and additional oil, if any, 0 to 20 weight percent. Initiator is 10% or less, usually about 5% or less, preferably 2% by weight or less and more preferably 1% or less. A preformed PBAE prepolymer that is derived from a first multifunctional acrylate and a multifunctional amine, is 2.5 to 45 weight percent, preferably 10 to 30 percent. The second water-soluble or dispersible mono- or multifunctional acrylate monomer is at 0.01 to 20 weight percent, preferably 1 to about 15 weight percent, more preferably 2 to 8% of the system. Acidic or basic (meth)acrylate monomer are each at 0.01 to 1 weight percent of the system.

The oil soluble or dispersible thermal free radical initiators can be an azo-based initiator or a peroxy initiator. Suitable free radical initiators may include peroxy initiators, azo initiators, peroxides, and compounds such as 2,2'-azobismethylbutyronitrile, dibenzoyl peroxide. More particularly, and without limitation, the free radical initiator can be selected from the group of initiators comprising an azo or peroxy initiator, such as peroxide, dialkyl peroxide, alkylperoxide, peroxyester, peroxycarbonate, peroxyketone and peroxydicarbonate, 2,2'-azobis (isobutylnitrile), 2,2'-azobis(2,4-dimethylpentanenitrile), 2,2'-azobis (2,4-dimethylvaleronitrile), 2,2'-azobis(2-methylpropanenitrile), 2,2'-azobis(2-methylbutyronitrile), 1,1'-azobis(cyclohexanecarbonitrile), 1,1'-azobis(cyanocyclohexane), benzoyl peroxide, decanoyl peroxide; lauroyl peroxide; benzoyl peroxide, di(n-propyl)peroxydicarbonate, di(sec-butyl) peroxydicarbonate, di(2-ethylhexyl)peroxydicarbonate, 1,1-dimethyl-3-hydroxybutyl peroxyneodecanoate, a-cumyl peroxyneoheptanoate, t-amyl peroxyneodecanoate, t-butyl peroxyneodecanoate, t-amyl peroxypivalate, t-butyl peroxypivalate, 2,5-dimethyl 2,5-di (2-ethylhexanoyl peroxy)hexane, t-amyl peroxy-2-ethyl-hexanoate, t-butyl peroxy-2-ethylhexanoate, t-butyl peroxyacetate, di-t-amyl peroxyacetate, t-butyl peroxide, di-t-amyl peroxide, 2,5-dimethyl-2,5-di-(t-butylperoxy)hexyne-3, cumene hydroperoxide, 1,1-di-(t-butylperoxy)-3,3,5-trimethyl-cyclohexane, 1,1-di-(t-butylperoxy)-cyclohexane, 1,1-di-(t-amylperoxy)-cyclohexane, ethyl-3,3 -di-(t-butylperoxy)-butyrate, t-amyl perbenzoate, t-butyl perbenzoate, ethyl 3,3-di-(t-amylperoxy)-butyrate, and the like.

In some embodiments, the bifunctional acrylate is Diethylene glycol diacrylate (SR230, Sartomer), Trifunctional trimethylolpropane triacrylate (SR351, Sartomer), Ethoxylated trimethylolpropane triacrylate (SR415, Sartomer), Ethoxylated trimethylolpropane triacrylate (SR454, Sartomer), pentaerythritol triacrylate (SR444, Sartomer), or pentaerythritol tetraacrylate (SR295, Sartomer). In certain embodiments, the polymer of the polymer wall may be further derived, at least in part, from at least one or more oil-soluble or oil-dispersible and/or water-soluble or water-dispersible free radical initiators. One or more free radical initiators can provide a source of free radicals upon activation.

Once the emulsion containing the first aqueous solution, the second aqueous solution and the oil phase is obtained, mix the third aqueous solution with the emulsion at a temperature of 25-70 °C. In one embodiment, this temperature is 50°C. Variations, such are less than three aqueous solutions, and changes in aqueous solution order or sequence of additions are within the contemplated scope of the invention.

Finally, the temperature of the resulted mixture is increased to an elevated temperature of 50-95 °C in a period of time between 30-120 mins, and hold at the elevated temperature for a period of time of 2-24 hours. Exemplary elevated temperatures can be 50 °C, 55 °C, 70 °C, 75 °C, 80 °C, 90 °C or 95°C. In one embodiment, the temperature of the resulted mixture is increased from 50°C to 75 °C in 60 mins and hold at 75 °C for 4 hours, and then increases to 95 °C in 60 mins and hold at 95 °C for 6 hours before cools down to room temperature.

In the above described method, polymerization reaction between the amine group of the multifunctional amine and the acrylate group of the multifunctional acrylate in the second aqueous solution occurs to form PBAE prepolymer. Polymerization reaction of the multifunctional (meth)acrylate in the oil phase occurs to form PAC delivery particle shell. Meanwhile, competing reactions, including the reaction between multifunctional (meth)acrylate and the amine moiety of the PBAE, the reaction between the acrylate group of PAC and the amine moiety of the PBAE, the reaction between the amine moiety in the PAC/PBAE backbone and between or among the multifunctional acrylate or the acrylate group of PBAE, occur to form hybrid PAC/PBAE. Hybrid PAC/PBAE is a copolymer of PAC and PBAE, and can include blended

polymers or copolymers derived from combinations of the above described various reaction pathways. PBAE can be formed in situ, sequentially or pre-formed separately in advance. The hybrid PAC/PBAE have both acrylate linkage and beta-amino esters linkage. These polymerization reactions may form an inner delivery particle shell of PAC and hybrid PAC/PBAE.

After a third aqueous solution containing the multifunctional acrylate is added into the emulsion containing the first aqueous solution, the second aqueous solution and the oil phase, the excess multifunctional acrylate in the third aqueous solution may further react with any primary or secondary amine moiety of PBAE prepolymer. In addition, the multifunctional acrylate may further crosslink PBAE in the aqueous solution and hybrid PAC/PBAE in the inner delivery particle shell. These reactions may lead to the formation of an outer delivery particle shell of PBAE, which crosslinks to the inner delivery particle shell of PAC and hybrid PAC/PBAE.

As produced in this invention, the PAC/PBAE delivery particle may have a single or dual shell structure including an inner shell or surface and an outer shell or surface. The PBAE in the outer shell or surface is covalently bonded with the PAC and hybrid PAC/PBAE in the inner shell or surface by the multifunctional acrylate in the third aqueous solution. Other crosslinking reactions include the reaction between acrylate moiety of PBAE with the amine moiety of hybrid PAC/PBAE, and the amine moiety of PBAE with or among acrylate of PAC or the acrylate moiety of hybrid PAC/PBAE. All these crosslinking reactions may contribute incorporating PBAE in the outer shell or surface into PAC and hybrid PAC/PBAE in an inner shell or surface.

In a method of making the PAC/PBAE delivery particles, the multifunctional acrylate may be not included in the second aqueous solution. If the multifunctional acrylate is not included in the second aqueous solution, PBAE prepolymer is not formed in the second aqueous solution. The self-polymerization of the multifunctional (meth)acrylate and the polymerization reaction between multifunctional (meth)acrylate in the oil phase and the multifunctional amine in the second aqueous solution occur to form an inner delivery particle shell of PAC/PBAE. If the multifunctional amine in the second aqueous phase is chitosan, chitin, gelatin, or other amine containing natural polymers, the emulsifier in the first aqueous phase is optional.

After the third aqueous solution containing a multifunctional acrylate is added into the emulsion, polymerization reaction between the multifunctional amine and the multifunctional acrylate occurs to form PBAE in the aqueous phase. As a result, hybrid PAC/PBAE is formed in the reactions between the multifunctional acrylate and the amine moiety in the PAC backbone, the acrylate group of PBAE and the amine moiety in the PAC backbone, the acrylate group of PAC and the amine moiety of the PBAE. These reactions may lead to the formation of a transitional delivery particle shell of hybrid PAC/PBAE.

In addition, further crosslinking reaction between the amine moiety of PBAE in the aqueous phase and the amine moiety of the hybrid PAC/PBAE in the transitional delivery particle shell by the multifunctional acrylate may lead to the formation of an outer delivery particle shell of PBAE.

As such, the method of making the PAC/PBAE delivery particles in the present invention may generate a PAC/PBAE delivery particle with an inner shell of PAC, a transitional shell of hybrid PAC/PBAE and an outer shell of PBAE.

The final product of the PAC/PBAE delivery particles is a slurry having oily medium-containing delivery particles encapsulated by PAC/PBAE polymer dispersed in the aqueous medium.

The delivery particle size distribution was measured using a light diffraction instrument. As measured, the PAC/PBAE delivery particles of the present invention have a particle size between 2-200 μm with a median particle size of 3-100 μm.

In order to test the properties of the PAC/PBAE delivery particles of the present invention, the PAC/PBAE delivery particles were subjected to a plurality of tests.

US 10,415,000 B2 and US 10,485,739 B2 disclose the methods of the measurements for the delivery particles and are hereby incorporated by reference.

Surface charge optionally can be built into the delivery particle shells with selection of monomers, or optionally added to the delivery particles with deposition aids or charged groups to improve the deposition of the delivery particles on substrates such as textiles, skin, hair, fibers, or other surfaces. The delivery particles can be over coated with the deposition aids as a post-encapsulation step such as by blending or mixing following or during the latter capsule formation steps. In certain embodiments the resultant delivery particles are cationic. Surface charge can also be advantageously employed to improve adhesion of delivery particles on surfaces such as foam or bedding material.

Deposition aids can include poly (acrylamide-co-diallyldimethylammonium chloride, poly (diallyldimethylammonium chloride, polyethylenimine, cationic polyamine, poly [(3-methyl-1-vinylimidazolium chloride)-*co*-(1-vinylpyrrolidone)], copolymer of acrylic acid and diallyldimethylammoni*um* chloride, cationic guar, guar gum, an organopolysiloxane such as described in US Patent Application Publication 2015/0030557, incorporated herein by reference. In a further embodiment, the above-described delivery particles can comprise a deposition aid, and in a further aspect the deposition aid coats the outer surface of the shell of the delivery particle. Deposition aids can be coated onto capsules or covalently bonded, employing functional groups to effect linkage as generally described in Universidade do Minho, WO 2006117702; Gross et al., US 20170296440; and Universidade do Minho, US 20080193761.

In a further aspect, the deposition aid can comprise a material selected from the group consisting of poly(meth)acrylate, poly(ethylene-maleic anhydride), polyamine, wax, polyvinylpyrrolidone, polyvinylpyrrolidone co-polymers, polyvinylpyrrolidone-ethyl acrylate, polyvinylpyrrolidone-vinyl acrylate, polyvinylpyrrolidone methacrylate, polyvinylpyrrolidone-vinyl acetate, polyvinyl acetal, polyvinyl butyral, polysiloxane, poly(propylene maleic anhydride), maleic anhydride derivatives, co-polymers of maleic anhydride derivatives, polyvinyl alcohol, styrene-butadiene latex, gelatin, gum Arabic, carboxymethyl cellulose, carboxymethyl hydroxyethyl cellulose, hydroxyethyl cellulose, other modified celluloses, sodium alginate, chitosan, casein, pectin, modified starch, polyvinyl acetal, polyvinyl butyral, polyvinyl methyl ether/maleic anhydride, polyvinyl pyrrolidone and its co polymers, poly(vinyl pyrrolidone/methacrylamidopropyl trimethyl ammonium chloride), polyvinylpyrrolidone/vinyl acetate, polyvinyl pyrrolidone/dimethylaminoethyl methacrylate, polyvinyl amines, polyvinyl formamides, polyallyl amines and copolymers of polyvinyl amines, polyvinyl formamides, and polyallyl amines and mixtures thereof.

In a yet further aspect, the deposition aid comprises a material selected from the group consisting of poly(meth)acrylates, poly(ethylene-maleic anhydride), polyamine, polyvinylpyrrolidone, polyvinylpyrrolidone-ethyl acrylate, polyvinylpyrrolidone-vinyl acrylate, polyvinylpyrrolidone methacrylate, polyvinylpyrrolidone-vinyl acetate, polyvinyl acetal, polysiloxane, poly(propylene maleic anhydride), maleic anhydride derivatives, co-polymers of maleic anhydride derivatives, polyvinyl alcohol, carboxymethyl cellulose, carboxymethyl hydroxyethyl cellulose, hydroxyethyl cellulose, polyvinyl methyl ether/maleic anhydride, polyvinylpyrrolidone/vinyl acetate, polyvinyl pyrrolidone/dimethylaminoethyl methacrylate, polyvinyl amines, polyvinyl formamides, polyallyl amines and copolymers of polyvinyl amines, polyvinyl formamides, and polyallyl amines and mixtures thereof.

Surface charge can also be advantageously adapted to create agglomerates to facilitate ease of filtration where a high solids, cake, or dry powder of delivery particles is desirable.

If desired, the delivery particles can be separated from the aqueous medium. The delivery particles can either be used as in an aqueous slurry, used as a dewatered cake, or used in dry powder form depending on the application.

The delivery particles of the invention can be incorporated dry, as an aqueous slurry, as a coating or as a gel into a variety of commercial products to yield novel and improved articles of manufacture, including incorporation into or onto foams, mattresses, bedding, cushions, added to cosmetics or to medical devices, incorporation into or onto packaging, dry wall, construction materials, heat sinks for electronics, cooling fluids, incorporation into insulation, used with lotions, incorporation into gels including gels for coating fabrics, automotive interiors, and other structures or articles, including clothing, footwear, personal protective equipment and any other article where use of the improved capsules of the invention is deemed desirable. The articles of manufacture can be selected from the group consisting of a soap, a surface cleaner, a laundry detergent, a fabric softener, a shampoo, a textile, a paper towel, an adhesive, a wipe, a diaper, a feminine hygiene product, a facial tissue, a pharmaceutical, a napkin, a deodorant, a foam, a pillow, a mattress, bedding, a cushion, a cosmetic, a medical device, an agricultural product, packaging, a cooling fluid, a wallboard, and an insulation.

The delivery particles protect and separate the core material, such as phase change material or fragrance or other core material or benefit agent, from the external environment. This facilitates design of distinct and improved articles of manufacture. The delivery particles facilitate improving flowability of encapsulated materials and enhancing ease of incorporation into or onto articles such as foams, gels, textiles, various cleaners, detergents or fabric softeners. The delivery particles can be used neat, or more often blended into coatings, gels or used as an aqueous slurry or blended into other articles to form new and improved articles of manufacture. For example, with phase change benefit agents, the delivery particles help preserve the repeated activity of the phase change material and retain the phase change material to prevent leakage or infusion into nearby components when isolation of the delivery particles is desired, yet promote eventual degradation of such encapsulates or portions of the articles of manufacture.

The shell of the composition according to the invention can achieve at least 10 % degradation or greater after as little as 28 days when tested according to test method OECD TG 301B. A surprising aspect is that capsules formed are not only tight capsules with low leakage but such capsules exhibit degradable properties in relatively short time periods. In embodiments delivery particles may have leakage values of below about 50% or below about 30%, as determined by the Leakage Test described in the TEST METHODS Section. Delivery particles according to the invention are of enhanced degradability as compared to capsules according to the prior art.

Method of making delivery particles

Delivery particles may be made according to known methods. Methods may be further adjusted to arrive at other desirable characteristics described herein, such as multiple shell structure.

For example, the present disclosure relates to a process of making a population of delivery particles comprising a core and a polymer shell encapsulating the core. The process may comprise following steps:

i. providing a first aqueous solution comprising an emulsifier and water;

ii. providing a second aqueous solution comprising a preformed PBAE polymer that contains free amino moieties or free acrylate moieties the preformed PBAE polymer comprises a reaction product by Aza-Michael addition reaction between a multifunctional amine, and a water-soluble or dispersible multifunctional acrylate,
iii. adding the first aqueous solution into the second aqueous solution under mixing to obtain a mixture of the first aqueous solution and the second aqueous solution;
iv. providing a first oil phase comprising the core material, a multifunctional (meth)acrylate, an acidic and/or a basic monofunctional (meth)acrylate;
v. providing a second oil phase comprising the core material and at least one oil soluble or dispersible thermal free radical initiator at elevated temperature for a period of time;
vi. adding the first oil phase into the second oil phase under mixing at elevated temperature for a period of time to obtain a mixture of the first and second oil phase;
vii. adding the mixture of the first oil phase and the second oil phase into the mixture of the first aqueous solution and the second aqueous solution, applying high shear agitation until a target particle size is reached to obtain an emulsion at a second temperature, the emulsion comprising an interface;
viii. providing a third aqueous solution comprising a second water-soluble or dispersible multifunctional acrylate, adding the third aqueous solution into the emulsion under mixing; and
ix. increasing a temperature to a third temperature in a second period of time and holding the temperature at the third temperature for a third period of time under mixing.

In embodiments, the process may comprise following steps:

i. providing a first aqueous solution comprising an emulsifier and water;
ii. providing a second aqueous solution comprising a multifunctional amine, a first multifunctional acrylate and water, and mixing the second aqueous solution at a first temperature for a first period of time;
iii. adding the first aqueous solution into the second aqueous solution under mixing to obtain a mixture of the first aqueous solution and the second aqueous solution;
iv. providing a first oil phase comprising the core material, a multifunctional (meth)acrylate, an acidic and/or a basic monofunctional (meth)acrylate;
v. providing a second oil phase comprising the core material and at least one oil soluble or dispersible thermal free radical initiator at elevated temperature for a period of time;
vi. adding the first oil phase into the second oil phase under mixing at a temperature from about 25 °C to about 70 °C from about 10 to about 360 minutes to obtain a mixture of the first and second oil phase;
vii. adding the mixture of the first oil phase and the second oil phase into the mixture of the first aqueous solution and the second aqueous solution, applying high shear agitation until a target drop size is reached to obtain an emulsion at a temperature from about 5 °C to about 55 °C;
viii. providing a third aqueous solution comprising a second multifunctional acrylate, adding the third aqueous solution into the emulsion under mixing; and
ix. increasing a temperature to from about 50 °C to about 95 °C in from about 30 to about 120 minutes and holding the temperature from about 2 to about 24 hours under mixing.

In embodiments, the process may comprise following steps:

i. providing a first aqueous solution comprising water and optionally an emulsifier;
ii. providing a second aqueous solution comprising a multifunctional amine and water;
iii. adding the first aqueous solution into the second aqueous solution under mixing to obtain a mixture of the first aqueous solution and the second aqueous solution;
iv. providing a first oil phase comprising the core material, a multifunctional (meth)acrylate, an acidic and/or a basic monofunctional (meth)acrylate;
v. providing a second oil phase comprising the core material and at least one oil soluble or dispersible thermal free radical initiator at elevated temperature for a period of time;
vi. adding the first oil phase into the second oil phase under mixing at a temperature from about 25 °C to about 70 °C from about 10 to about 360 minutes to obtain a mixture of the first and second oil phase;
vii. adding the mixture of the first oil phase and the second oil phase into the mixture of the first aqueous solution and the second aqueous solution, applying high shear agitation until a target particle size is reached to obtain an emulsion at a temperature from about 5 °C to about 55 °C;
viii. providing a third aqueous solution comprising a multifunctional acrylate, adding the third aqueous solution into the emulsion under mixing; and
ix. increasing a temperature to from about 50 °C to about 95 °C in from about 30 to about 120 minutes and holding

the temperature from about 2 to about 24 hours under mixing.

In embodiments, the process may comprise following steps:

i. providing a first aqueous solution comprising an emulsifier and water;
ii. providing a second aqueous solution comprising a multifunctional amine, a multifunctional acrylate and water, and mixing the second aqueous solution at a first temperature for a first period of time;
iii. adding the first aqueous solution into the second aqueous solution under mixing to obtain a mixture of the first aqueous solution and the second aqueous solution;
iv. providing a first oil phase comprising the core material, a multifunctional (meth)acrylate, an acidic and/or a basic monofunctional (meth)acrylate;
v. providing a second oil phase comprising the core material and at least one oil soluble or dispersible thermal free radical initiator at elevated temperature for a period of time;
vi. adding the first oil phase into the second oil phase under mixing at a temperature from about 25 °C to about 70 °C from about 10 to about 360 minutes to obtain a mixture of the first and second oil phase;
vii. adding the mixture of the first oil phase and the second oil phase into the mixture of the first aqueous solution and the second aqueous solution, applying high shear agitation until a target particle size is reached to obtain an emulsion at a temperature from about 5 °C to about 55 °C;
viii. increasing a temperature to from about 50 °C to about 95 °C in from about 30 to about 120 minutes and holding the temperature from about 2 to about 24 hours under mixing.

In embodiments, the process may comprise following steps:

i. providing a first aqueous solution comprising an emulsifier and water;
ii. providing a second aqueous solution comprising a multifunctional amine, a first multifunctional acrylate and water, and mixing the second aqueous solution at a first temperature for a first period of time;
iii. providing a first oil phase comprising the core material, a multifunctional (meth)acrylate, an acidic and/or a basic monofunctional (meth)acrylate;
iv. providing a second oil phase comprising the core material and at least one oil soluble or dispersible thermal free radical initiator at elevated temperature for a period of time;
v. adding the first oil phase into the second oil phase under mixing at a temperature from about 25 °C to about 70 °C from about 10 to about 360 minutes to obtain a mixture of the first and second oil phase;
vi. adding the mixture of the first oil phase and the second oil phase into the first aqueous solution, applying high shear agitation at a temperature from about 5 °C to about 55 °C until a target drop size is reached to obtain a first emulsion;
vii. adding the second aqueous solution into the first emulsion under mixing to obtain a second emulsion;
viii. providing a third aqueous solution comprising a second multifunctional acrylate, and adding the third aqueous solution into the second emulsion under mixing;
ix. increasing a temperature to from about 50 °C to about 95 °C in from about 30 to about 120 minutes and holding the temperature from about 2 to about 24 hours under mixing.

In embodiments, the process may comprise following steps:

i. providing a first aqueous solution comprising water and optionally an emulsifier;
ii. providing a second aqueous solution comprising a multifunctional amine and water;
iii. providing a first oil phase comprising the core material, a multifunctional (meth)acrylate, an acidic and/or a basic monofunctional (meth)acrylate;
iv. providing a second oil phase comprising the core material and at least one oil soluble or dispersible thermal free radical initiator at elevated temperature for a period of time;
v. adding the first oil phase into the second oil phase under mixing at elevated temperature for a period of time to obtain a mixture of the first and second oil phase;
vi. adding the mixture of the first oil phase and the second oil phase into into the first aqueous solution, applying high shear agitation at a temperature from about 5 °C to about 55 °C until a target drop size is reached to obtain a first emulsion;
vii. adding the second aqueous solution into the first emulsion under mixing to obtain a second emulsion;
viii. providing a third aqueous solution comprising a multifunctional acrylate, and adding the third aqueous solution into the second emulsion under mixing;
ix. increasing a temperature to a temperature from about 50 °C to about 95 °C in from about 30 to about 120 minutes

and hold the temperature for from about 2 to about 24 hours under mixing.

In embodiments, the process may comprise following steps:

i. providing a first aqueous solution comprising an emulsifier and water;
ii. providing a second aqueous solution comprising a multifunctional amine, a multifunctional acrylate and water, and mixing the second aqueous solution at a first temperature for a first period of time;
iii. providing a first oil phase comprising the core material, a multifunctional (meth)acrylate, an acidic and/or a basic monofunctional (meth)acrylate;
iv. providing a second oil phase comprising the core material and at least one oil soluble or dispersible thermal free radical initiator at a temperature from about 25 °C to about 70 °C for from about 10 to about 360 minutes;
v. adding the first oil phase into the second oil phase under mixing at elevated temperature for a period of time to obtain a mixture of the first and second oil phase;
vi. adding the mixture of the first oil phase and the second oil phase into the first aqueous solution, applying high shear agitation at a temperature from about 5 °C to about 55 °C until a target drop size is reached to obtain a first emulsion;
vii. adding the second aqueous solution into the first emulsion under mixing to obtain a second emulsion; and
viii. increasing a temperature to from about 50 °C to about 95 °C in from about 30 to about 120 minutes and holding the temperature from about 2 to about 24 hours under mixing.

In embodiments, the process may comprise following steps:

i. providing a first aqueous solution comprising an emulsifier and water;
ii. providing a first oil phase comprising the core material, a multifunctional (meth)acrylate, an acidic and/or a basic monofunctional (meth)acrylate;
iii. providing a second oil phase comprising the core material and at least one oil soluble or dispersible thermal free radical initiator at elevated temperature for a sufficient period of time to form free radicals;
iv. adding the first oil phase into the second oil phase under mixing at a temperature from about 25 °C to about 70 °C for from about 10 to about 360 minutes to obtain a mixture of the first and second oil phase;
v. providing a third oil phase comprising a preformed PBAE polymer that contains free acrylate moieties. The preformed PBAE polymer comprising a reaction product by Aza-Michael addition reaction between a multifunctional amine, and a multifunctional acrylate;
vi. adding the third oil phase into the mixture of the first oil phase and the second oil phase and mixing for from about 10 to about 360 minutes;
vii. adding the mixture of the first oil phase, the second oil phase and the third oil phase into the first aqueous solution, applying high shear agitation until a target drop size is reached to obtain an emulsion at a second temperature, the emulsion comprising an interface;
viii. increasing a temperature to from about 50 °C to about 95 °C in from about 30 to about 120 minutes and holding the temperature from about 2 to about 24 hours under mixing.

In embodiments, the multifunctional amine is selected from diethylenetriamine, ethylenediamine, tetraethylenepentaamine, pentaethylenehexamine, polyethylenimine, chitosan, chitin, gelatin, arginine, lysine, ornithine, nisin, or histidine, the water-soluble or dispersible multifunctional acrylate is selected from diethylene glycol diacrylate, triethylene glycol diacrylate, tetraethylene glycol diacrylate, poly(ethylene glycol) diacrylate, trifunctional trimethylolpropane triacrylate, ethoxylated trimethylolpropane triacrylate, or a combination thereof, and the multifunctional (meth)acrylate monomer and /or oligomer is selected from group consisting of tri-functional (meth)acrylate, tetra-functional (meth)acrylate, penta-functional (meth)acrylate, hexa-functional (meth)acrylate, hepta-functional (meth)acrylate, and mixtures thereof.

In embodiments, the preformed PBAE prepolymer contains free amino moieties that react with the multifunctional (meth)acrylate in the oil phase, at the interface via Aza-Michael Addition reaction. In embodiments, the preformed PBAE prepolymer further comprises free (meth)acrylate moieties that react with the multifunctional (meth)acrylate via free radical polymerization in the oil phase or at the interface.

In embodiments, the preformed PBAE prepolymer further comprises free amino moieties that react with the multifunctional acrylate in the water phase via Aza-Michael Addition reaction.

Exemplary synthesis methods that can form delivery particles according the present disclosure are further described in EXAMPLES section below.

PROCESS OF MAKING CONSUMER PRODUCT COMPOSITIONS

The present application relates to processes for making any of the compositions described herein. The process of making a composition may comprise the step of combining a benefit agent delivery particle as described herein with an adjunct material which may be a consumer product adjunct material as described herein.

The particles may be combined with such one or more adjunct materials such as consumer product adjuncts materials when the particles are in one or more forms, including a slurry form, neat particle form, and/or spray dried particle form. The particles may be combined with adjunct materials such as consumer product adjuncts materials by methods that include mixing and/or spraying.

The compositions of the present disclosure can be formulated into any suitable form and prepared by any process chosen by the formulator. The particles and adjunct materials may be combined in a batch process, in a circulation loop process, and/or by an in-line mixing process. Suitable equipment for use in the processes disclosed herein may include continuous stirred tank reactors, homogenizers, turbine agitators, recirculating pumps, paddle mixers, plough shear mixers, ribbon blenders, vertical axis granulators and drum mixers, both in batch and, where available, in continuous process configurations, spray dryers, and extruders.

HAIR CARE COMPOSITIONS

The delivery particle of the current invention can be used in hair care compositions to provide one or more benefits, including freshness, malodor removal, softness and styling. The hair care compositions of the present invention can be in different forms. Non-limiting examples of said forms are shampoos, conditioning shampoos, pet shampoo, leave-on treatments, sprays, liquids, pastes, Newtonian or non-Newtonian fluids, gels, and sols.

The hair care composition preferably comprises delivery particles at least comprising one benefit agent at a level where upon directed use, promotes one or more benefits without detriment to the hair. Such benefit agent may comprise a perfume, an essential oil, a silicone, a wax and mixtures thereof. The perfume may comprise a single perfume raw material or a mixture of perfume raw materials. Examples of essential oils are argan oil, lavender oil, peppermint oil, rosemary oil, thyme oil, cedarwood oil, lemongrass oil, ylang-ylang oil and mixtures thereof.

In one embodiment of the present invention, said hair care composition comprises between about 0.01wt% to about 15wt% of at least one benefit agent encapsulated in a delivery particle. In another embodiment, said hair care composition comprises between about 0.05wt% to about 8wt% of at least one benefit agent encapsulated. In another embodiment, said hair care composition comprises between about 0.1 wt% to about 5wt% of at least one benefit agent encapsulated.

In addition to at least one delivery particle, the hair care compositions of the present invention may also include detersive surfactants, aqueous carriers, shampoo gel matrixes, and other additional ingredients.

Detersive Surfactant

The hair care composition comprises one or more detersive surfactants, which provides cleaning performance to the composition. The one or more detersive surfactants in turn may comprise an anionic surfactant, amphoteric or zwitterionic surfactants, or mixtures thereof. Various examples and descriptions of detersive surfactants are set forth in U.S. Patent No. 6,649,155; U.S. Patent Application Publication No. 2008/0317698; and U.S. Patent Application Publication No. 2008/0206355, which are incorporated herein by reference in their entirety.

The concentration of the detersive surfactant component in the hair care composition should be sufficient to provide the desired cleaning and lather performance, and generally ranges from 2 wt% to about 50 wt%, from about 5 wt% to about 30 wt%, from about 8 wt% to about 25 wt%, from about 10 wt% to about 20 wt%, about 5 wt%, about 10 wt%, about 12 wt%, about 15 wt%, about 17 wt%, about 18 wt%, or about 20 wt%.

Anionic surfactants suitable for use in the compositions are the alkyl and alkyl ether sulfates. Other suitable anionic surfactants are the water-soluble salts of organic, sulfuric acid reaction products. Still other suitable anionic surfactants are the reaction products of fatty acids esterified with isethionic acid and neutralized with sodium hydroxide. Other similar anionic surfactants are described in U.S. Patent Nos. 2,486,921; 2,486,922; and 2,396,278, which are incorporated herein by reference in their entirety.

Exemplary anionic surfactants for use in the hair care composition include ammonium lauryl sulfate, ammonium laureth sulfate, triethylamine lauryl sulfate, triethylamine laureth sulfate, triethanolamine lauryl sulfate, triethanolamine laureth sulfate, monoethanolamine lauryl sulfate, monoethanolamine laureth sulfate, diethanolamine lauryl sulfate, diethanolamine laureth sulfate, lauric monoglyceride sodium sulfate, sodium lauryl sulfate, sodium laureth sulfate, potassium lauryl sulfate, potassium laureth sulfate, sodium lauryl sarcosinate, sodium lauroyl sarcosinate, lauryl sarcosine, cocoyl sarcosine, ammonium cocoyl sulfate, ammonium lauroyl sulfate, sodium cocoyl sulfate, sodium lauroyl sulfate, potassium cocoyl sulfate, potassium lauryl sulfate, triethanolamine lauryl sulfate, triethanolamine lauryl sulfate, monoethanolamine cocoyl sulfate, monoethanolamine lauryl sulfate, sodium tridecyl benzene sulfonate, sodium dodecyl benzene sulfonate, sodium cocoyl isethionate and combinations thereof. In a further embodiment, the anionic surfactant is sodium lauryl sulfate or sodium laureth sulfate.

Suitable amphoteric or zwitterionic surfactants for use in the hair care composition herein include those which are known for use in shampoo or other personal care cleansing. Concentrations of such amphoteric surfactants range from about 0.5 wt% to about 20 wt%, and from about 1 wt% to about 10 wt%. Non limiting examples of suitable zwitterionic or amphoteric surfactants are described in U.S. Patent Nos. 5,104,646 and 5,106,609, which are incorporated herein by reference in their entirety.

Amphoteric detersive surfactants suitable for use in the hair care composition include those surfactants broadly described as derivatives of aliphatic secondary and tertiary amines in which the aliphatic radical can be straight or branched chain and wherein one of the aliphatic substituents contains from about 8 to about 18 carbon atoms and one contains an anionic group such as carboxy, sulfonate, sulfate, phosphate, or phosphonate. Exemplary amphoteric detersive surfactants for use in the present hair care composition include cocoamphoacetate, cocoamphodiacetate, lauroamphoacetate, lauroamphodiacetate, and mixtures thereof.

Zwitterionic detersive surfactants suitable for use in the hair care composition include those surfactants broadly described as derivatives of aliphatic quaternaryammonium, phosphonium, and sulfonium compounds, in which the aliphatic radicals can be straight or branched chain, and wherein one of the aliphatic substituents contains from about 8 to about 18 carbon atoms and one contains an anionic group such as carboxy, sulfonate, sulfate, phosphate or phosphonate. In another embodiment, zwitterionics such as betaines are selected.

Non limiting examples of other anionic, zwitterionic, amphoteric or optional additional surfactants suitable for use in the hair care composition are described in McCutcheon's, Emulsifiers and Detergents, 1989 Annual, published by M. C. Publishing Co., and U.S. Patent Nos. 3,929,678, 2,658,072; 2,438,091; 2,528,378, which are incorporated herein by reference in their entirety.

The hair care composition may also comprise a shampoo gel matrix, an aqueous carrier, and other additional ingredients described herein.

Aqueous Carrier

The hair care composition comprises a first aqueous carrier. The level and species of the carrier are selected according to the compatibility with other components, and other desired characteristic of the product. Accordingly, the formulations of the hair care composition can be in the form of pourable liquids (under ambient conditions). Such compositions will therefore typically comprise a first aqueous carrier, which is present at a level of at least 20 wt%, from about 20 wt% to about 95 wt%, or from about 60 wt% to about 85 wt%. The first aqueous carrier may comprise water, or a miscible mixture of water and organic solvent, and in one aspect may comprise water with minimal or no significant concentrations of organic solvent, except as otherwise incidentally incorporated into the composition as minor ingredients of other components.

Shampoo Gel Matrix

In one embodiment, the hair care composition described herein may comprise a shampoo gel matrix. The shampoo gel matrix comprises (i) from about 0.1% to about 20% of one or more fatty alcohols, alternative from about 0.5% to about 14%, alternatively from about 1% to about 10%, alternatively from about 6% to about 8%, by weight of the shampoo gel matrix; (ii) from about 0.1% to about 10% of one or more shampoo gel matrix surfactants, by weight of the shampoo gel matrix; and (iii) from about 20% to about 95% of an aqueous carrier, alternatively from about 60% to about 85% by weight of the shampoo gel matrix.

The fatty alcohols useful herein are those having from about 10 to about 40 carbon atoms, from about 12 to about 22 carbon atoms, from about 16 to about 22 carbon atoms, or about 16 to about 18 carbon atoms. These fatty alcohols can be straight or branched chain alcohols and can be saturated or unsaturated. Nonlimiting examples of fatty alcohols include, cetyl alcohol, stearyl alcohol, behenyl alcohol, and mixtures thereof. Mixtures of cetyl and stearyl alcohol in a ratio of from about 20:80 to about 80:20 are suitable.

The shampoo gel matrix surfactants may be any of the detersive surfactants described in section "A" herein.

The aqueous carrier may comprise water, or a miscible mixture of water and organic solvent, and in one aspect may comprise water with minimal or no significant concentrations of organic solvent, except as otherwise incidentally incorporated into the composition as minor ingredients of other components.

The aqueous carrier useful herein includes water and water solutions of lower alkyl alcohols and polyhydric alcohols. The lower alkyl alcohols useful herein are monohydric alcohols having 1 to 6 carbons, in one aspect, ethanol and isopropanol. Exemplary polyhydric alcohols useful herein include propylene glycol, hexylene glycol, glycerin, and propane diol.

Additional Ingredients

Silicone Conditioning Agent

The compositions of the present invention may contain one or more silicone conditioning agents. Examples of the silicones include dimethicones, dimethiconols, cyclic silicones, methylphenyl polysiloxane, and modified silicones with various functional groups such as amino groups, quaternary ammonium salt groups, aliphatic groups, alcohol groups, carboxylic acid groups, ether groups, sugar or polysaccharide groups, fluorine-modified alkyl groups, alkoxy groups, or combinations of such groups. Such silicones may be soluble or insoluble in the aqueous (or non-aqueous) product carrier. In the case of insoluble liquid silicones, the silicones can be in an emulsified form with droplet size of about 10 nm to about 30 micrometers Other solid or semi-solid conditioning agents may be present in the composition including high melting temperature fatty alcohols, acids, esters, amides or oligomers from unsaturated esters, alcohols, amides. The oligomeric esters may be the result of oligomerization of naturally-occurring unsaturated glyceride esters. Such solid or semi-solid conditioning agents may be added or present as mixtures with organic oils.

Nonionic Polymers

The hair care composition of the present invention may also further comprise a nonionic polymer. According to an embodiment, the conditioning agent for use in the hair care composition of the present invention may include a polyalkylene glycol polymer. For example, polyalkylene glycols having a molecular weight of more than about 1000 are useful herein. Useful are those having the following general formula:

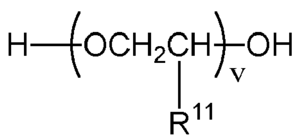

wherein R11 is selected from the group consisting of H, methyl, and mixtures thereof; and v is the number of ethoxy units. The polyalkylene glycols, such as polyethylene glycols, can be included in the hair care compositions of the present invention at a level of from about 0.001 wt.% to about 10 wt.%. In an embodiment, the polyethylene glycol is present in an amount up to about 5 wt.% based on the weight of the composition. Polyethylene glycol polymers useful herein are PEG-2M (also known as Polyox WSR® N-10, which is available from Union Carbide and as PEG-2,000); PEG-5M (also known as Polyox WSR® N-35 and Polyox WSR® N-80, available from Union Carbide and as PEG-5,000 and Polyethylene Glycol 300,000); PEG-7M (also known as Polyox WSR® N-750 available from Union Carbide); PEG-9M (also known as Polyox WSR® N-3333 available from Union Carbide); and PEG-14 M (also known as Polyox WSR® N-3000 available from Union Carbide).

Organic Conditioning Materials

The conditioning agent of the compositions of the present invention may also comprise at least one organic conditioning material such as oil or wax, either alone or in combination with other conditioning agents, such as the silicones described above. The organic material can be non-polymeric, oligomeric or polymeric. It may be in the form of oil or wax and may be added in the formulation neat or in a pre-emulsified form. Some non-limiting examples of organic conditioning materials include, but are not limited to: i) hydrocarbon oils; ii) polyolefins, iii) fatty esters, iv) fluorinated conditioning compounds, v) fatty alcohols, vi) alkyl glucosides and alkyl glucoside derivatives; vii) quaternary ammonium compounds; viii) polyethylene glycols and polypropylene glycols having a molecular weight of up to about 2,000,000 including those with CTFA names PEG-200, PEG-400, PEG-600, PEG-1000, PEG-2M, PEG-7M, PEG-14M, PEG-45M and mixtures thereof.

Deposition Aids

The hair care compositions of the present invention may further comprise a deposition aid, such as a cationic polymer. Cationic polymers useful herein are those having an average molecular weight of at least about 5,000, alternatively from about 10,000 to about 10 million, and alternatively from about 100,000 to about 2 million.

Suitable cationic polymers include, for example, copolymers of vinyl monomers having cationic amine or quaternary ammonium functionalities with water-soluble spacer monomers such as acrylamide, methacrylamide, alkyl and dialkyl acrylamides, alkyl and dialkyl methacrylamides, alkyl acrylate, alkyl methacrylate, vinyl caprolactone, and vinyl

pyrrolidone. Other suitable spacer monomers include vinyl esters, vinyl alcohol (made by hydrolysis of polyvinyl acetate), maleic anhydride, propylene glycol, and ethylene glycol. Other suitable cationic polymers useful herein include, for example, cationic celluloses, cationic starches, and cationic guar gums.

The cationic polymer can be included in the hair care compositions of the present invention at a level of from about 0.001 wt.% to about 10 wt.%. In one embodiment, the cationic polymer is present in an amount up to about 5 wt% based on the weight of the composition.

Hair Care Benefit Agents

In an embodiment, the hair care composition further comprises one or more additional benefit agents. The benefit agents comprise a material selected from the group consisting of antidandruff agents, anti-fungal agents, anti-itch agents, anti-bacterial agents, anti-microbial agents, moisturization agents, anti-oxidants, vitamins, lipid soluble vitamins, chelants, perfumes, brighteners, enzymes, sensates, attractants, dyes, pigments, bleaches, and mixtures thereof.

Rheology Modifier / Suspending Agents

In one embodiment, the rinse-off hair care composition comprises a rheology modifier. The rheology modifier increases the substantivity and stability of the composition, improves feel and consumer's use experience (e.g. non-dripping, spreadability, etc). Any suitable rheology modifier can be used. In an embodiment, the hair care composition may comprise from about 0.05% to about 10% of a rheology modifier, in a further embodiment, from about 0.1% to about 10% of a rheology modifier, in yet a further embodiment, from about 0.5% to about 2 % of a rheology modifier, in a further embodiment, from about 0.7% to about 2% of a rheology modifier, and in a further embodiment from about 1% to about 1.5% of a rheology modifier. In an embodiment, the rheology modifier may be a polyacrylamide thickener. In an embodiment, the rheology modifier may be a polymeric rheology modifier.

In an embodiment, the composition of the present invention may comprise suspending agents including crystalline suspending agents which can be categorized as acyl derivatives, long chain amine oxides, and mixtures thereof. These suspending agents are described in U.S. Pat. No. 4,741,855. These suspending agents include ethylene glycol esters of fatty acids in one aspect having from about 16 to about 22 carbon atoms. In embodiments, useful suspending agents include ethylene glycol stearates, both mono and distearate, but in one aspect, the distearate containing less than about 7% of the mono stearate. Other suitable suspending agents include alkanol amides of fatty acids, having from about 16 to about 22 carbon atoms, or even about 16 to 18 carbon atoms, examples of which include stearic monoethanolamide, stearic diethanolamide, stearic monoisopropanolamide and stearic monoethanolamide stearate. Other long chain acyl derivatives include long chain esters of long chain fatty acids (e.g., stearyl stearate, cetyl palmitate, etc.); long chain esters of long chain alkanol amides (e.g., stearamide diethanolamide distearate, stearamide monoethanolamide stearate); and glyceryl esters (e.g., glyceryl distearate, trihydroxystearin, tribehenin) a commercial example of which is Thixin® R available from Rheox, Inc. Long chain acyl derivatives, ethylene glycol esters of long chain carboxylic acids, long chain amine oxides, and alkanol amides of long chain carboxylic acids in addition to the materials listed above may be used as suspending agents. Other long chain acyl derivatives suitable for use as suspending agents include N,N-dihydrocarbyl amido benzoic acid and soluble salts thereof (e.g., Na, K), particularly N,N-di(hydrogenated) C16, C18 and tallow amido benzoic acid species of this family, which are commercially available from Stepan Company (Northfield, Ill., USA). Examples of suitable long chain amine oxides for use as suspending agents include alkyl dimethyl amine oxides, e.g., stearyl dimethyl amine oxide. Other suitable suspending agents include primary amines having a fatty alkyl moiety having at least about 16 carbon atoms, examples of which include palmitamine or stearamine, and secondary amines having two fatty alkyl moieties each having at least about 12 carbon atoms, examples of which include dipalmitoylamine or di(hydrogenated tallow)amine. Still other suitable suspending agents include di(hydrogenated tallow)phthalic acid amide, and crosslinked maleic anhydride-methyl vinyl ether copolymer.

PERSONAL CLEANSING COMPOSITIONS

The delivery particle of the current invention can be used in personal cleansing compositions to provide one or more benefits, including freshness and/or softeness. The personal cleansing care compositions of the present invention can be in different forms. Non-limiting examples of said forms are: bar soap, body wash, moisturizing body wash, shower gels, skin cleansers, cleansing milks, in shower body moisturizer, shaving preparations, cleansing compositions used in conjunction with a disposable cleansing cloth, sprays, liquids, pastes, Newtonian or non-Newtonian fluids, gels, and sols.

The personal cleansing composition preferably comprises delivery particles at least comprising one benefit agent at a level where upon directed use, promotes one or more benefits. In one embodiment of the present invention, said personal cleansing composition comprises between about 0.01wt% to about 15wt% of at least one benefit agent

encapsulated in said delivery particle. In another embodiment, said personal cleansing composition comprises between about 0.05% to about 8% of at least one benefit agent encapsulated. In another embodiment, said personal cleansing composition comprises between about 0.1% to about 5% of at least one benefit agent encapsulated.

In addition to at least one delivery particle, the personal cleansing compositions of the present invention may also include additional ingredients.

Personal cleansing compositions can be multi-phase or single phase. While the components for personal cleansing compositions will be discussed below as being multi-phase for simplicity, the components for each phase could also be used in a single phase. A personal cleansing composition can comprise a cleansing phase and a benefit phase. The cleansing phase and the benefit phase can be blended. The cleansing phase and the benefit phase can also be patterned (e.g. striped and/or marbled). In embodiments, the cleansing phase may comprise the delivery particle. In embodiments, the benefit phase may comprise the delivery particle.

Cleansing Phase

A personal cleansing composition can comprise from about 50% to about 99.5%, by weight of the composition, of a cleansing phase. A cleansing phase can include a surfactant. The personal care composition can further comprise from 2% to 20%, by weight of the rinse-off personal care composition, of a surfactant. Surfactants can comprise anionic surfactants, nonionic surfactants, amphoteric surfactants, zwitterionic surfactants, cationic surfactants, or mixtures thereof. The personal care composition can include at least one anionic surfactant. A personal care composition can also comprise, for example, an anionic surfactant, amphoteric surfactant, and a zwitterionic surfactant. Suitable amphoteric or zwitterionic surfactants, for example, can include those described in U.S. Patent No. 5,104,646 and U.S. Patent No. 5,106,609.

Anionic surfactants suitable for use in the cleansing phase of the present compositions include alkyl and alkyl ether sulfates. These materials have the respective formula ROSO3M and RO(C2H4O)xSO3M, wherein R is alkyl or alkenyl of from about 8 to about 24 carbon atoms, wherein x is about 1 to about 10, and M is a water-soluble cation such as ammonium, sodium, potassium, or triethanolamine. The alkyl ether sulfates are typically made as condensation products of ethylene oxide and monohydric alcohols having from about 8 to about 24 carbon atoms. R may have from about 10 to about 18 carbon atoms in both the alkyl and alkyl ether sulfates. The alcohols can be derived from fats, e.g., coconut oil or tallow, or can be synthetic. Lauryl alcohol and straight chain alcohols derived from coconut oil may be used. Such alcohols may be reacted with about 1 or about 3 to about 10 or about 5 molar proportions of ethylene oxide. The resulting mixture of molecular species may have, for example, an average of 3 moles of ethylene oxide per mole of alcohol, is sulfated and neutralized.

Other suitable anionic surfactants include water-soluble salts of the organic, sulfuric acid reaction products of the general formula [R1-SO3-M], wherein R1 is chosen from the group consisting of a straight or branched chain, saturated aliphatic hydrocarbon radical having from about 8 to about 24, or about 10 to about 18, carbon atoms; and M is a cation. Suitable examples are the salts of an organic sulfuric acid reaction product of a hydrocarbon of the methane series, including iso-, neo-, ineso-, and n-paraffins, having about 8 to about 24 carbon atoms, preferably about 10 to about 18 carbon atoms and a sulfonating agent, e.g., SO3, H2SO4, oleum, obtained according to known sulfonation methods, including bleaching and hydrolysis. Preferred are alkali metal and ammonium sulfonated C10-18 n-paraffins.

Suitable anionic surfactants for use in the cleansing phase include ammonium lauryl sulfate, ammonium laureth sulfate, triethylamine lauryl sulfate, triethylamine laureth sulfate, triethanolamine lauryl sulfate, triethanolamine laureth sulfate, monoethanolamine lauryl sulfate, monoethanolamine laureth sulfate, diethanolamine lauryl sulfate, diethanolamine laureth sulfate, lauric monoglyceride sodium sulfate, sodium lauryl sulfate, sodium laureth sulfate, potassium laureth sulfate, sodium lauryl sarcosinate, sodium lauroyl sarcosinate, lauryl sarcosine, cocoyl sarcosine, ammonium cocoyl sulfate, ammonium lauroyl sulfate, sodium cocoyl sulfate, sodium lauroyl sulfate, potassium cocoyl sulfate, potassium lauryl sulfate, monoethanolamine cocoyl sulfate, sodium tridecyl benzene sulfonate, sodium dodecyl benzene sulfonate, and combinations thereof.

Anionic surfactants with branched alkyl chains such as sodium trideceth sulfate, for example, may be employed. Mixtures of anionic surfactants can also be used.

Amphoteric surfactants can include those that can be broadly described as derivatives of aliphatic secondary and tertiary amines in which an aliphatic radical can be straight or branched chain and wherein an aliphatic substituent can contain from about 8 to about 18 carbon atoms such that one carbon atom can contain an anionic water solubilizing group, e.g., carboxy, sulfonate, sulfate, phosphate, or phosphonate. Examples of compounds falling within this definition can be sodium 3-dodecyl-aminopropionate, sodium 3-dodecylaminopropane sulfonate, sodium lauryl sarcosinate, N-alkyltaurines such as the one prepared by reacting dodecylamine with sodium isethionate according to the teaching of U.S. Pat. No. 2,658,072, N-higher alkyl aspartic acids such as those produced according to the teaching of U.S. Pat. No. 2,438,091, and products described in U.S. Pat. No. 2,528,378. Other examples of amphoteric surfactants can include sodium lauroamphoacetate, sodium cocoamphoactetate, disodium lauroamphoacetate disodium cocodiamphoacetate,

and mixtures thereof. Amphoacetates and diamphoacetates can also be used.

Zwitterionic surfactants suitable for use as cleansing surfactant in the structured aqueous cleansing phase include those that are broadly described as derivatives of aliphatic quaternary ammonium, phosphonium, and sulfonium compounds, in which the aliphatic radicals can be straight or branched chain, and wherein one of the aliphatic substituents contains from about 8 to about 18 carbon atoms and one contains an anionic group, e.g., carboxy, sulfonate, sulfate, phosphate, or phosphonate.

Other zwitterionic surfactants suitable for use in the cleansing phase include betaines, including high alkyl betaines such as coco dimethyl carboxymethyl betaine, cocoamidopropyl betaine, cocobetaine, lauryl amidopropyl betaine, oleyl betaine, lauryl dimethyl carboxymethyl betaine, lauryl dimethyl alphacarboxyethyl betaine, cetyl dimethyl carboxymethyl betaine, lauryl bis-(2-hydroxyethyl) carboxymethyl betaine, stearyl bis-(2-hydroxypropyl) carboxymethyl betaine, oleyl dimethyl gammacarboxypropyl betaine, and lauryl bis-(2-hydroxypropyl)alphacarboxyethyl betaine. The sulfobetaines may be represented by coco dimethyl sulfopropyl betaine, stearyl dimethyl sulfopropyl betaine, lauryl dimethyl sulfoethyl betaine, lauryl bis-(2-hydroxyethyl) sulfopropyl betaine and the like; amidobetaines and amidosulfobetaines, wherein the RCONH(CH2)3 radical is attached to the nitrogen atom of the betaine are also useful in the present compositions.

Amphoacetates and diamphoacetates can also be used. Non-limiting examples of suitable amphoacetates and diamphoacetates include sodium lauroamphoacetate, sodium cocoamphoactetate, disodium lauroamphoacetate, and disodium cocodiamphoacetate.

Cationic surfactants can also be used in the cleansing phase and may represent from 2% to about 5%, by weight of the cleansing phase.

Suitable nonionic surfactants for use in structured aqueous cleansing phase include condensation products of alkylene oxide groups (hydrophilic in nature) with an organic hydrophobic compound, which may be aliphatic or alkyl aromatic in nature.

Other suitable surfactants or cosurfactants that can generally be used in a cleansing phase for a rinse-off personal care composition are described in McCutcheon's: Detergents and Emulsifiers North American Edition (Allured Publishing Corporation 1947) (1986), McCutcheon's, Functional Materials North American Edition (Allured Publishing Corporation 1973) (1992) and U.S. Patent No. 3,929,678 (filed Aug. 1, 1974).

The cleansing phase can include a structuring surfactant. Such a structuring surfactant can be included from 2% to about 20%, by weight of the personal care composition; from about 3% to about 15%, by weight of the personal care composition; or from about 5% to about 10%, by weight of the personal care composition. Such a structuring surfactant can include sodium trideceth(n) sulfate, hereinafter STnS, wherein n defines the average moles of ethoxylation. n can range, for example, from about 0 to about 3; n can range from about 0.5 to about 2.7; from about 1.1 to about 2.5; from about 1.8 to about 2.2; or n can be about 2. When n is less than 3, STnS can provide improved stability, improved compatibility of benefit agents within the rinse-off personal care compositions, and/or increased mildness of the rinse-off personal care compositions, such described benefits of STnS are disclosed in U.S. Patent Application Pub. No. 2012/0009285.

The personal care composition can further comprise from about 2% to 20%, by weight of the personal care composition, of a cosurfactant. Cosurfactants can comprise amphoteric surfactants, zwitterionic surfactants, or mixtures thereof. Examples of these types of surfactant are discussed above.

The personal care composition can also comprise a water-soluble cationic polymer. The water-soluble cationic polymer can be present from about 0.001 to about 3 percent by weight of the personal care composition. The water-soluble cationic polymer can also be present from about 0.05 to about 2 percent by weight of the personal care composition. The water-soluble cationic polymer can also be present from about 0.1 to about 1 by weight of the personal care composition. The polymer may be in one or more phases as a deposition aid for the benefit agents described herein. Suitable cationic deposition polymers for use in the compositions of the present invention contain, for example, cationic nitrogen-containing moieties such as quaternary ammonium or cationic protonated amino moieties. The cationic protonated amines can be primary, secondary, or tertiary amines depending upon the particular species and the selected pH of the personal care composition.

Nonlimiting examples of cationic deposition polymers for use in compositions include polysaccharide polymers, such as cationic cellulose derivatives. The cationic cellulose polymers can be, for example, the salts of hydroxyethyl cellulose reacted with trimethyl ammonium substituted epoxide, referred to in the industry (CTFA) as Polyquaternium 10 which are available from Amerchol Corp. (Edison, N.J., USA) in their Polymer KG, JR and LR series of polymers. The water-soluble cationic polymer comprises, for example, KG-30M. Other suitable cationic deposition polymers include cationic guar gum derivatives, such as guar hydroxypropyltrimonium chloride, specific examples of which include the Jaguar series (preferably Jaguar C-17) commercially available from Rhodia Inc., and N-Hance polymer series commercially available from Ashland.

The water-soluble cationic polymer can comprise, for example, a cationic guar. In one example, the cationic guar comprises guar hydroxypropyltrimonium chloride. The guar hydroxypropyltrimonium chloride can comprise, for

example, N-hanceTM CG-17 Cationic Guar. The cationic guar can be, for example, selected from a group consisting of N-hanceTM 3196, Jaguar C-500, Jaguar C-17, and a combination thereof. Deposition polymers can have a cationic charge density from about 0.8 meq/g to about 2.0 meq/g or from about 1.0 meq/g to about 1.5 meq/g, or about 0.96 meq/g.

The water-soluble cationic polymer can also comprise synthetic polyacrylamides. Examples of suitable synthetic polyacrylamides include polyquaternium 76 and Polymethylene-bis-acrylamide methacrylamido propyltrimethyl ammonium chloride (PAMMAPTAC, AM MAPTAC ratio 88:12. In one example, the water-soluble cationic polymer comprises PAM/MAPTAC.

A cleansing phase of a personal care composition can also include an associative polymer. Such associative polymer can be a crosslinked, alkali swellable, associative polymer comprising acidic monomers and associative monomers with hydrophobic end groups, whereby the associative polymer comprises a percentage hydrophobic modification and a hydrophobic side chain comprising alkyl functional groups. Without intending to be limited by theory, it is believed the acidic monomers can contribute to an ability of the associative polymer to swell in water upon neutralization of acidic groups; and associative monomers anchor the associative polymer into structured surfactant hydrophobic domains, e.g., lamellae, to confer structure to the surfactant phase and keep the associative polymer from collapsing and losing effectiveness in a presence of an electrolyte.

The crosslinked, associative polymer can comprise a percentage hydrophobic modification, which is a mole percentage of monomers expressed as a percentage of a total number of all monomers in a polymer backbone, including both acidic and other non-acidic monomers. Percentage hydrophobic modification of the associative polymer, hereafter %HM, can be determined by the ratio of monomers added during synthesis, or by analytical techniques such as proton nuclear magnetic resonance (NMR). Associative alkyl side chains can comprise, for example, butyl, propyl, stearyl, steareth, cetyl, lauryl, laureth, octyl, behenyl, beheneth, steareth, or other linear, branched, saturated, or unsaturated alkyl or alketh hydrocarbon side chains. The acidic monomer can comprise any acid functional group, for example sulfate, sulfonate, carboxylate, phosphonate, or phosphate or mixtures of acid groups. The acidic monomer can comprise, for example, a carboxylate, alternatively the acidic monomer is an acrylate, including acrylic acid and/or methacrylic acid. The acidic monomer comprises a polymerizable structure, e.g., vinyl functionality. Mixtures of acidic monomers, for example acrylic acid and methacrylic acid monomer mixtures, are useful.

The associative monomer can comprise a hydrophobic end group and a polymerizable component, e.g., vinyl, which can be attached. The hydrophobic end group can be attached to the polymerizable component, hence to the polymer chain, by different means but can be attached by an ether or ester or amide functionality, such as an alkyl acrylate or a vinyl alkanoate monomer. The hydrophobic end group can also be separated from the chain, for example, by an alkoxy ligand such as an alkyl ether. The associative monomer can be, for example, an alkyl ester, an alkyl (meth)acrylate, where (meth)acrylate is understood to mean either methyl acrylate or acrylate, or mixtures of the two.

The hydrophobic end group of the associative polymer can be incompatible with the aqueous phase of the composition and can associate with lathering surfactant hydrophobe components. Without intending to be limited by theory, it is believed that longer alkyl chains of structuring polymer hydrophobe end groups can increase incompatibility with the aqueous phase to enhance structure, whereas somewhat shorter alkyl chains having carbon numbers closely resembling lathering surfactant hydrophobes (e.g., 12 to 14 carbons) or multiples thereof (for bilayers, e.g.) can also be effective. An ideal range of hydrophobic end group carbon numbers combined with an optimal percentage of hydrophobic monomers expressed as a percentage of the polymer backbone can provide increased structure to the lathering, structured surfactant composition at low levels of polymer structurant.

Other optional additives can be included in the cleansing phase, including for example an emulsifier (e.g., nonionic emulsifier) and electrolytes. Suitable emulsifiers and electrolytes are described in U.S. Patent Application Serial No. 13/157,665.

Personal Care Composition Benefit Phase

As noted herein, personal care compositions can include a benefit phase. The composition may comprise from about 0.1 % to about 50%, by weight of the composition, of a benefit phase. The benefit phase can be hydrophobic and/or anhydrous. The benefit phase can also be substantially free of or free of surfactant. In particular, the benefit phase can comprise from about 0.1% to about 50%, by weight of the rinse-off personal care composition, of a benefit agent. The benefit phase can include, for example, from about 0.5% to about 20%, by weight of the rinse-off personal care composition, of a benefit agent.

A benefit phase can have a particle size of about 4 to about 500 μm, from about 5 to about 300μm, from about 6 to about 100 μm, or from about 10 to about 50 μm. The particle size is measured in neat product under a differential interference contrast optical microscope with a 10x objective lens. The particle size distribution is counted manually. All benefit phase particles are assumed as uniform spheres in this application. For irregular shaped benefit phase particles, the longest axis is used as the diameter for the particle size distribution counting. The number weighted average of all lipid particles is defined as the average lipid particle size. This measurement can also be accomplished with a computer

algorithm.

A benefit phase can have a viscosity as measured by a standard rheometer, such as a Brookfield R/S plus. A sample of 2.5 mL is measured with a spindle C75-1 at a shear rate of 2 s-1 at 25°C. A benefit phase can generally have a viscosity of about 200 cP to about 15,000cP.

A benefit agent can include a liquid benefit agent. A liquid benefit agent is considered liquid if that is its natural state at room temperature (i.e. 23°C). A liquid benefit agent can have a viscosity of less than about 1000 cP, less than about 800 cP, or less than about 600 cP, and can be measured with a standard rheometer.

The benefit agent may also be non-liquid. Some examples of non-liquid benefit agents include hydrocarbons. Non-limiting examples of hydrocarbons suitable for use as non-liquid benefit agents herein can include petrolatum, microcrystalline wax, polyalkanes, polyolefins, and combinations thereof.

Other suitable benefit agents are described in U.S. Patent Application Publication No. 2012/0009285.

The benefit phase may also comprise a crystalline hydrophobic ethylene copolymer. The ethylene copolymers are random copolymers and may be present from about 0.01 % to about 5 % by weight of the personal care composition. The crystalline hydrophobic ethylene copolymer may be present from about 0.05 % to about 2 % by weight of the personal care composition. As another example, the crystalline hydrophobic ethylene copolymer may be present from about 0.1 % to about 1.5 % by weight of the personal care composition.

Additional Ingredients

Additional ingredients can also be added to the personal care composition for treatment of the skin and/or hair, or to modify the aesthetics of the personal care composition as is the case with perfumes, colorants, dyes or the like. Materials useful in products herein can be categorized or described by their cosmetic and/or therapeutic benefit or their postulated mode of action or function. However, it can be understood that actives and other materials useful herein can, in some instances, provide more than one cosmetic and/or therapeutic benefit or function or operate via more than one mode of action. Therefore, classifications herein can be made for convenience and cannot be intended to limit an ingredient to particularly stated application or applications listed. A precise nature of these additional materials, and levels of incorporation thereof, will depend on the physical form of the composition and the nature of the cleansing operation for which it is to be used. The additional materials can usually be formulated at about 6% or less, about 5% or less, about 4% or less, about 3% or less, about 2% or less, about 1% or less, about 0.5% or less, about 0.25% or less, about 0.1% or less, about 0.01% or less, or about 0.005% or less of the rinse-off personal care composition.

To further improve stability under stressful conditions such as high temperature and vibration, densities of separate phases can be adjusted such that they can be substantially equal. To achieve this, low density microspheres can be added to one or more phases of the rinse-off personal care composition. Examples of rinse-off personal care compositions that comprise low density microspheres are described in a patent application published on May 13, 2004 under U.S. Patent Publication No. 2004/0092415A1 entitled "Striped Liquid Personal Cleansing Compositions Containing A Cleansing Phase and A Separate Phase with Improved Stability," filed on Oct. 31, 2003 by Focht, et al.

Other non-limiting ingredients that can be used in the personal care composition of the present invention can comprise an optional benefit component that can be selected from the group consisting of thickening agents; preservatives; antimicrobials; fragrances; chelators (e.g. such as those described in U.S. Pat. No. 5,487,884 issued to Bisset, et al.); sequestrants; vitamins (e.g. Retinol); vitamin derivatives (e.g. tocophenyl actetate, niacinamide, panthenol); sunscreens; desquamation actives (e.g. such as those described in U.S. Pat. No. 5,681,852 and 5,652,228 issued to Bisset); anti-wrinkle/ anti-atrophy actives (e.g. N-acetyl derivatives, thiols, hydroxyl acids, phenol); anti-oxidants (e.g. ascorbic acid derivatives, tocophenol) skin soothing agents/skin healing agents (e.g. panthenoic acid derivatives, aloe vera, allantoin); skin lightening agents (e.g. kojic acid, arbutin, ascorbic acid derivatives) skin tanning agents (e.g. dihydroxyacteone); anti-acne medicaments; essential oils; sensates; pigments; colorants; pearlescent agents; interference pigments (e.g such as those disclosed in U.S. Pat. No. 6,395,691 issued to Liang Sheng Tsaur, U.S. Pat. No. 6,645,511 issued to Aronson, et al., U.S. Pat. No. 6,759,376 issued to Zhang, et al, U.S. Pat. No. 6,780,826 issued to Zhang, et al.) particles (e.g. talc, kolin, mica, smectite clay, cellulose powder, polysiloxane, silicas, carbonates, titanium dioxide, polyethylene beads) hydrophobically modified non-platelet particles (e.g. hydrophobically modified titanium dioxide and other materials described in a commonly owned, patent application published on Aug. 17, 2006 under Publication No. 2006/0182699A, entitled "Personal Care Compositions Containing Hydrophobically Modified Non-platelet particle filed on Feb. 15, 2005 by Taylor, et al.) and mixtures thereof. The multiphase personal care composition can comprise from about 0.1% to about 4%, by weight of the rinse-off personal care composition, of hydrophobically modified titanium dioxide. Other such suitable examples of such skin actives are described in U.S. Patent Application Serial No. 13/157,665.

SHAVE PREPARATIONS

The delivery particle of the current invention can be used in shave preparations to provide one or more benefits, including freshness and/or cooling. The shave preparations of the present invention can be in different forms. Non-limiting examples of said forms are: shaving creams, shaving gels, aerosol shaving gels, shaving soaps, aerosol shaving foams, liquids, pastes, Newtonian or non-Newtonian fluids, gels, and sols.

The shave preparation preferably comprises at least one benefit agent encapsulated in said delivery particle at a level where upon directed use, promotes one or more benefits. In one embodiment of the present invention, said shave preparation comprises between about 0.01% to about 15% of at least one benefit agent encapsulated in said delivery particle. In another embodiment, said shave preparation comprises between about 0.05% to about 8% of at least one benefit agent encapsulated. In another embodiment, said shave preparation comprises between about 0.1% to about 5% of at least one benefit agent encapsulated.

In addition to at least one delivery particle, the shave preparations of the present invention may also include lathering surfactants, carriers, adjunct ingredients, and other additional ingredients.

Lathering Surfactants

The shave preparations can comprise one or more lathering surfactants and a carrier such as water, at a total level of from about 60% to about 99.99%. A lathering surfactant defined herein as surfactant, which when combined with water and mechanically agitated generates a foam or lather. Preferably, these surfactants or combinations of surfactants should be mild, which means that these surfactants provide sufficient cleansing or detersive benefits but do not overly dry the skin or hair while still being able to produce a lather.

A wide variety of lathering surfactants are useful herein and include those selected from the group consisting of anionic lathering surfactants, nonionic lather surfactants, amphoteric lathering surfactants, and mixtures thereof. Generally, the lathering surfactants are fairly water-soluble. When used in the composition, at least about 4% of the lathering surfactants have a HLB value greater than about ten. Examples of such surfactants are found in and U.S. Pat. 5,624,666. Cationic surfactants can also be used as optional components, provided they do not negatively impact the overall lathering characteristics of the required lathering surfactants.

Concentrations of these surfactant are from about 10% to about 20%, alternatively from about 5% to about 25%, and alternatively from 2% to about 60% by weight of the composition.

Anionic lathering surfactants useful in the compositions of the present invention are disclosed in McCutcheon's, Detergents and Emulsifiers, North American edition (1986), published by allured Publishing Corporation; McCutcheon's, Functional Materials, North American Edition (1992); and U.S. Patent No. 3,929,678. A wide variety of anionic lathering surfactants are useful herein. Non-limiting examples of anionic lathering surfactants include those selected from the group consisting of sarcosinates, sulfates, sulfonates, isethionates, taurates, phosphates, lactylates, glutamates, and mixtures thereof.

Other anionic materials useful herein are soaps (i.e., alkali metal salts, e.g., sodium or potassium salts) of fatty acids, typically having from about 8 to about 24 carbon atoms, preferably from about 10 to about 20 carbon atoms, monoalkyl, dialkyl, and trialkylphosphate salts, alkanoyl sarcosinates corresponding to the formula RCON(CH3)CH2CH2CO2M wherein R is alkyl or alkenyl of about 10 to about 20 carbon atoms, and M is a water-soluble cation such as ammonium, sodium, potassium and alkanolamine (e.g., triethanolamine). Also useful are taurates which are based on taurine, which is also known as 2-aminoethanesulfonic acid, and glutamates, especially those having carbon chains between C8 and C16.

Suitable amphoteric or zwitterionic detersive surfactants for use in the compositions herein include those which are known for use in hair care or other personal care cleansing. Concentration of such amphoteric detersive surfactants is from about 1% to about 10%, alternatively from about 0.5 % to about 20% by weight of the composition. Non-limiting examples of suitable zwitterionic or amphoteric surfactants are described in U.S. Patent Nos. 5,104,646 and 5,106,609.

Nonionic lathering surfactants for use in the compositions of the present invention are disclosed in McCutcheon's, Detergents and Emulsifiers, North American edition (1986), published by allured Publishing Corporation; and McCutcheon's, Functional Materials, North American Edition (1992); both of which are incorporated by reference herein in their entirety. Nonionic lathering surfactants useful herein include those selected from the group consisting of alkyl glucosides, alkyl polyglucosides, polyhydroxy fatty acid amides, alkoxylated fatty acid esters, lathering sucrose esters, amine oxides, and mixtures thereof.

Preferred lathering surfactants for use herein are the following, wherein the anionic lathering surfactant is selected from the group consisting of ammonium lauroyl sarcosinate, sodium trideceth sulfate, sodium lauroyl sarcosinate, sodium myristoyl sarcosinate, ammonium laureth sulfate, sodium laureth sulfate, ammonium lauryl sulfate, sodium lauryl sulfate, ammonium cocoyl isethionate, sodium cocoyl isethionate, sodium lauroyl isethionate, sodium cetyl sulfate, sodium lauroyl lactylate, triethanolamine lauroyl lactylate, and mixtures thereof; wherein the nonionic lathering surfactant

is selected from the group consisting of lauramine oxide, cocoamine oxide, decyl polyglucose, lauryl polyglucose, sucrose cocoate, C12-14 glucosamides, sucrose laurate, and mixtures thereof; and wherein the amphoteric lathering surfactant is selected from the group consisting of disodium lauroamphodiacetate, sodium lauroamphoacetate, cetyl dimethyl betaine, cocoamidopropyl betaine, cocoamidopropyl hydroxy sultaine, and mixtures thereof.

One suitable lathering surfactant is a polyglyceryl fatty ester. In one embodiment the polyglyceryl fatty ester surfactant has the formula:

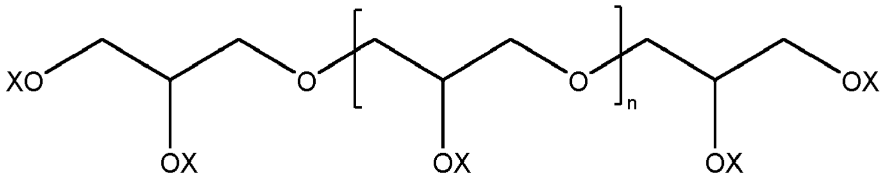

wherein n is 1 to 10, and X is a hydrogen atom or a long chain acyl group derived from a C12-22 fatty acid or an N-fatty acyl-neutral amino acid, provided that at least one X is a long chain acyl group and no more than three X's are long chain acyl groups. In one embodiment, the polyglyceryl fatty ester surfactant is selected from the group consisting of: polyglyceryl-10 oleate, polyglyceryl-6 stearate, polyglyceryl-10 stearate, polyglyceryl-8 dipalmitate, polyglyceryl-10 dipalmitate, polyglyceryl-10 behenate, and polyglyceryl-12 trilaurate.

Carriers

The shave preparation of the present invention can also comprise a carrier. In one embodiment the carrier comprises water. The carrier is preferably dermatologically acceptable, meaning that the carrier is suitable for topical application to the keratinous tissue, has good aesthetic properties, is compatible with the actives of the present invention and any other components, and will not cause any safety or toxicity concerns. In one embodiment, the shave preparation comprises from about 50% to about 99.99%, preferably from about 60% to about 99.9%, more preferably from about 70% to about 98%, and even more preferably from about 80% to about 95% of the carrier by weight of the composition.

Adjunct Ingredients

Lubricants

In one embodiment, said shave preparation comprises at least one lubricant selected from: a lubricious water-soluble polymer; a water insoluble particle, a hydrogel forming polymer, and a mixture thereof.

The lubricious water-soluble polymer will generally have a molecular weight greater between about 300,000 and 15,000,000 daltons, preferably more than about one million daltons, and will include a sufficient number of hydrophilic moieties or substituents on the polymer chain to render the polymer water-soluble. The polymer may be a homopolymer, copolymer or terpolymer. Examples of suitable lubricious water-soluble polymers include polyethylene oxide, polyvinylpyrrolidone, and polyacrylamide. A preferred lubricious water-soluble polymer comprises polyethylene oxide, and more particularly a polyethylene oxide with a molecular weight of about 0.5 to about 5 million daltons. Examples of suitable polyethylene oxides PEG-23M, PEG-45M, and PEG-90M. The lubricious water-soluble polymer can be at a level of about 0.005% to about 3%, preferably about 0.01% to about 1%, by weight.

The water insoluble particles may include inorganic particles or organic polymer particles. Examples of inorganic particles include titanium dioxide, silicas, silicates and glass beads, with glass beads being preferred. Examples of organic polymer particles include polytetrafluoroethylene particles, polyethylene particles, polypropylene particles, polyurethane particles, polyamide particles, or mixtures of two or more of such particles.

The hydrogel-forming polymer is a highly hydrophilic polymer that, in water, forms organized three-dimensional domains of approximately nanometer scale. The hydrogel-forming polymer generally has a molecular weight greater than about one million daltons (although lower molecular weights are possible) and typically is at least partially or lightly crosslinked and may be at least partially water insoluble, but it also includes a sufficient number of hydrophilic moieties so as to enable the polymer to trap or bind a substantial amount of water within the polymer matrix and thereby form three-dimensional domains. Generally, the hydrogel-forming polymer will be included in the shaving composition in an amount of about 0.0005% to about 3%, or about 0.001% to about 0.5%, or about 0.002% to about 0.1%, by weight.

The term "water dispersible", as used herein, means that a substance is either substantially dispersible or soluble in water. The water dispersible surface active agent is preferably one that is capable of forming a lather, such as one or more of the optional lathering surfactants described in section 5 below (including but not limited to a soap, an interrupted soap, a detergent, an anionic surfactant, a non-ionic surfactant or a mixture of one or more of these.)

Polar Solvents

In one embodiment, the carrier comprises a polar solvent. The level of polar solvent can be from about 1% to about 20%, or from about 5 % to about 10%. Polar solvents useful herein include polyhydric alcohols such as ,3-butylene glycol, propane diol, ethylene glycol, diethylene glycol, sorbitol, and other sugars which are in liquid form at ambient temperature glycerin, sorbitol, propylene glycol, butylene glycol, pentylene glycol, hexylene glycol, ethoxylated glucose, 1,2-hexane diol, hexanetriol, dipropylene glycol, erythritol, trehalose, diglycerin, xylitol, maltitol, maltose, glucose, fructose, sodium chondroitin sulfate, sodium hyaluronate, sodium adenosine phosphate, sodium lactate, pyrrolidone carbonate, glucosamine, cyclodextrin, and mixtures thereof. Polyols such as those containing from 2 to about 6 carbon atoms and from 2 to about 6 hydroxy groups are preferred (e.g., 1,3-propanediol, ethylene glycol, glycerin, and 1,2-propanediol) can also be used. The most preferred are Butylene, Pentylene or Hexylene Glycol and mixtures thereof.

Salicylic Acid

The shave preparation of the present invention may comprise a salicylic acid compound, its esters, its salts, or combinations thereof. In the compositions of the present invention, the salicylic acid compound preferably comprises from about 0.1% to about 5%, preferably from about 0.2% to about 2%, and more preferably from about 0.5% to about 2%, by weight of the composition, of salicylic acid.

Other Adjunct Ingredients

The compositions of the present invention may contain a variety of other ingredients that are conventionally used in given product types provided that they do not unacceptably alter the benefits of the invention. These ingredients should be included in a safe and effective amount for a shave preparation for application to skin.

Conditioning Agents

The compositions of the present invention may comprise a conditioning agent selected from the group consisting of humectants, moisturizers, or skin conditioners, each can be present at a level of from about 0.01% to about 40%, more preferably from about 0.1% to about 30%, and even more preferably from about 0.5% to about 15% by weight of the composition. These materials include, but are not limited to, guanidine; urea; glycolic acid and glycolate salts (e.g. ammonium and quaternary alkyl ammonium); lactic acid and lactate salts (e.g., ammonium and quaternary alkyl ammonium); aloe vera in any of its variety of forms (e.g., aloe vera gel); polyhydroxy compounds such as sorbitol, mannitol, glycerol, hexanetriol, butanetriol, propylene glycol, butylene glycol, hexylene glycol and the like; polyethylene glycols; sugars (e.g., melibiose) and starches; sugar and starch derivatives (e.g., alkoxylated glucose, fructose, sucrose, etc.); hyaluronic acid; lactamide monoethanolamine; acetamide monoethanolamine; sucrose polyester; petrolatum; and mixtures thereof.

Suitable moisturizers, also referred to in the present invention as humectants, include urea, guanidine, glycolic acid and glycolate salts (e.g. ammonium and quaternary alkyl ammonium), lactic acid and lactate salts (e.g. ammonium and quaternary alkyl ammonium), aloe vera in any of its variety of forms (e.g. aloe vera gel), polyhydroxy alcohols (such as sorbitol, glycerol, hexanetriol, propylene glycol, hexylene glycol and the like), polyethylene glycol, sugars and starches, sugar and starch derivatives (e.g. alkoxylated glucose), hyaluronic acid, lactamide monoethanolamine, acetamide monoethanolamine, and mixtures thereof.

Thickening Agents (including thickeners and gelling agents)

The compositions of the present invention can comprise one or more thickening agents, preferably from about 0.05% to about 10%, more preferably from about 0.1% to about 5%, and even more preferably from about 0.25% to about 4%, by weight of the composition. Nonlimiting classes of thickening agents include those selected from the group consisting of: Carboxylic Acid Polymers (crosslinked compounds containing one or more monomers derived from acrylic acid, substituted acrylic acids, and salts and esters of these acrylic acids and the substituted acrylic acids, wherein the crosslinking agent contains two or more carbon-carbon double bonds and is derived from a polyhydric alcohol); crosslinked polyacrylate polymers (including both cationic and nonionic polymers, such as described in U. S. Patent No. 5,100,660; 4,849,484; 4,835,206; 4,628,078; 4,599,379, and EP 228,868); polymeric sulfonic acid (such as copolymers of acryloyldimethyltaurate and vinylpyrrolidone) and hydrophobically modified polymeric sulfonic acid (such as crosspolymers of acryloyldimethyltaurate and beheneth-25 methacrylate); polyacrylamide polymers (such as nonionic polyacrylamide polymers including substituted branched or unbranched polymers such as polyacrylamide and isoparaffin and laureth-7 and multiblock copolymers of acrylamides and substituted acrylamides with acrylic acids and substituted

acrylic acids); polysaccharides (nonlimiting examples of polysaccharide gelling agents include those selected from the group consisting of cellulose, carboxymethyl hydroxyethylcellulose, cellulose acetate propionate carboxylate, hydroxyethylcellulose, hydroxyethyl ethylcellulose, hydroxypropylcellulose, hydroxypropyl methylcellulose, methyl hydroxyethylcellulose, microcrystalline cellulose, sodium cellulose sulfate, and mixtures thereof); gums (i.e. gum agents such as acacia, agar, algin, alginic acid, ammonium alginate, amylopectin, calcium alginate, calcium carrageenan, carnitine, carrageenan, dextrin, gelatin, gellan gum, guar gum, guar hydroxypropyltrimonium chloride, hectorite, hyaluronic acid, hydrated silica, hydroxypropyl chitosan, hydroxypropyl guar, karaya gum, kelp, locust bean gum, natto gum, potassium alginate, potassium carrageenan, propylene glycol alginate, sclerotium gum, sodium carboxymethyl dextran, sodium carrageenan, tragacanth gum, xanthan gum, and mixtures thereof); and crystalline, hydroxyl-containing fatty acids, fatty esters or fatty waxes (such as microfibrous bacterial cellulose structurants as disclosed in U.S. Patent Nos. 6,967,027 to Heux et al.; 5,207,826 to Westland et al.; 4,487,634 to Turbak et al.; 4,373,702 to Turbak et al. and 4,863,565 to Johnson et al., U.S. Patent Publ. No. 2007/0027108 to Yang et al.)

Compositional pH

The shave preparation of the present invention preferably has a pH of less than about 9, more preferably less than about 7. In one embodiment the composition has a pH of less than about 5, or less than about 4. In one preferred embodiment the composition has a pH range of from about 2.5 to about 4.5. Suitable lathering surfactants for use at pH levels below about 4 can be selected from the group consisting of alkyl sulfonates, pareth sulfonates, sulfobetaines, alkylhydroxysultaines, alkylglucosides and mixtures thereof.

FABRIC CARE COMPOSITIONS

Fabric care compositions of the present invention may include additional adjunct ingredients that include: bleach activators, surfactants, builders, chelating agents, dye transfer inhibiting agents, dispersants, enzymes, and enzyme stabilizers, catalytic metal complexes, polymeric dispersing agents, clay and soil removal/anti-redeposition agents, suds suppressors, dyes, additional perfumes and perfume delivery systems, structure elasticizing agents, fabric softeners, carriers, hydrotropes, processing aids, structurants, anti-agglomeration agents, coatings, formaldehyde scavengers and/or pigments. Other variants of Applicants' compositions do not contain one or more of the following adjuncts materials: bleach activators, surfactants, builders, chelating agents, dye transfer inhibiting agents, dispersants, enzymes, and enzyme stabilizers, catalytic metal complexes, polymeric dispersing agents, clay and soil removal/anti-redeposition agents, suds suppressors, dyes, additional perfumes and perfume delivery systems, structure elasticizing agents, fabric softeners, carriers, hydrotropes, processing aids, structurants, anti-agglomeration agents, coatings, formaldehyde scavengers, malodor reduction materials and/or pigments. The precise nature of these additional components, and levels of incorporation thereof, will depend on the physical form of the composition and the nature of the operation for which it is to be used. However, when one or more adjuncts are present, such one or more adjuncts may be present as detailed below. The following is a non-limiting list of suitable additional adjuncts.

Deposition Aid

The fabric care composition may comprise from about 0.01% to about 10%, from about 0.05 to about 5%, or from about 0.15 to about 3% of a deposition aid. The deposition aid may be a cationic or amphoteric polymer. The deposition aid may be a cationic polymer. Cationic polymers in general and their method of manufacture are known in the literature. The cationic polymer may have a cationic charge density of from about 0.005 to about 23 meq/g, from about 0.01 to about 12 meq/g, or from about 0.1 to about 7 meq/g, at the pH of the composition. For amine-containing polymers, wherein the charge density depends on the pH of the composition, charge density is measured at the intended use pH of the product. Such pH will generally range from about 2 to about 11, more generally from about 2.5 to about 9.5. Charge density is calculated by dividing the number of net charges per repeating unit by the molecular weight of the repeating unit. The positive charges may be located on the backbone of the polymers and/or the side chains of polymers.

The weight-average molecular weight of the polymer may be from about 500 Daltons to about 5,000,000 Daltons, or from about 1,000 Daltons to about 2,000,000 Daltons, or from about 2,500 Daltons to about 1,500,000 Daltons, as determined by size exclusion chromatography relative to polyethylene oxide standards with RI detection. The weight-average molecular weight of the cationic polymer may be from about 500 Daltons to about 37,500 Daltons.

Surfactants: Surfactants utilized can be of the anionic, nonionic, zwitterionic, ampholytic or cationic type or can comprise compatible mixtures of these types, as described above in relation to HAIR CARE, PERSONAL CARE, and SHAVE CARE Compositions. Anionic and nonionic surfactants are typically employed if the fabric care product is a laundry detergent. On the other hand, cationic surfactants are typically employed if the fabric care product is a fabric

softener. In addition to the anionic surfactant, the fabric care compositions of the present invention may further contain a nonionic surfactant. The compositions of the present invention can contain up to about 30%, alternatively from about 0.01% to about 20%, more alternatively from about 0.1% to about 10%, by weight of the composition, of a nonionic surfactant. The nonionic surfactant may comprise an ethoxylated nonionic surfactant. Suitable for use herein are the ethoxylated alcohols and ethoxylated alkyl phenols of the formula $R(OC2H4)n$ OH, wherein R is selected from the group consisting of aliphatic hydrocarbon radicals containing from about 8 to about 20 carbon atoms and alkyl phenyl radicals in which the alkyl groups contain from about 8 to about 12 carbon atoms, and the average value of n is from about 5 to about 15.

The fabric care compositions of the present invention may contain up to about 30%, alternatively from about 0.01% to about 20%, more alternatively from about 0.1% to about 20%, by weight of the composition, of a cationic surfactant. For the purposes of the present invention, cationic surfactants include those which can deliver fabric care benefits. Non-limiting examples of useful cationic surfactants include: fatty amines; quaternary ammonium surfactants; and imidazoline quat materials.

Non-limiting examples of fabric softening actives are N, N-bis(stearoyl-oxy-ethyl) N,N-dimethylammonium chloride; N,N-bis(tallowoyl-oxy-ethyl) N,N-dimethylammonium chloride, N,N-bis(stearoyl-oxy-ethyl)N-(2 hydroxyethyl)N-methyl ammonium methyl sulfate; 1, 2 di (stearoyl-oxy) 3 trimethyl ammoniumpropane chloride; dialkylenedimethylammonium salts such as dicanoladimethylammonium chloride, di(hard)tallowdimethylammonium chloride dicanoladimethylammonium methyl sulfate; 1-methyl-1-stearoylamidoethyl-2-stearoylimidazolinium methyl sulfate; 1-tallowylamidoethyl-2-tallowylimidazoline; N,N"-dialkyldiethylenetriamine; the reaction product of N-(2-hydroxyethyl)-1,2-ethylenediamine or N-(2-hydroxyisopropyl)-1,2-ethylenediamine with glycolic acid, esterified with fatty acid, where the fatty acid is (hydrogenated) tallow fatty acid, palm fatty acid, hydrogenated palm fatty acid, oleic acid, rapeseed fatty acid, hydrogenated rapeseed fatty acid; polyglycerol esters (PGEs), oily sugar derivatives, and wax emulsions and a mixture of the above.

It will be understood that combinations of softener actives disclosed above are suitable for use herein.

Builders

The compositions may also contain from about 0.1% to 80% by weight of a builder. Compositions in liquid form generally contain from about 1% to 10% by weight of the builder component. Compositions in granular form generally contain from about 1% to 50% by weight of the builder component. Detergent builders are well known in the art and can contain, for example, phosphate salts as well as various organic and inorganic nonphosphorus builders. Water-soluble, nonphosphorus organic builders useful herein include the various alkali metal, ammonium and substituted ammonium polyacetates, carboxylates, polycarboxylates and polyhydroxy sulfonates. Examples of polyacetate and polycarboxylate builders are the sodium, potassium, lithium, ammonium and substituted ammonium salts of ethylene diamine tetraacetic acid, nitrilotriacetic acid, oxydisuccinic acid, mellitic acid, benzene polycarboxylic acids, and citric acid. Other polycarboxylate builders are the oxydisuccinates and the ether carboxylate builder compositions comprising a combination of tartrate monosuccinate and tartrate disuccinate. Builders for use in liquid detergents include citric acid. Suitable nonphosphorus, inorganic builders include the silicates, aluminosilicates, borates and carbonates, such as sodium and potassium carbonate, bicarbonate, sesquicarbonate, tetraborate decahydrate, and silicates having a weight ratio of $SiO2$ to alkali metal oxide of from about 0.5 to about 4.0, or from about 1.0 to about 2.4. Also useful are aluminosilicates including zeolites.

Dispersants

The compositions may contain from about 0.1%, to about 10%, by weight of dispersants. Suitable water-soluble organic materials are the homo- or co-polymeric acids or their salts, in which the polycarboxylic acid may contain at least two carboxyl radicals separated from each other by not more than two carbon atoms. The dispersants may also be alkoxylated derivatives of polyamines, and/or quaternized derivatives.

Enzymes

The compositions may contain one or more detergent enzymes which provide cleaning performance and/or fabric care benefits. Examples of suitable enzymes include hemicellulases, peroxidases, proteases, cellulases, xylanases, lipases, phospholipases, esterases, cutinases, pectinases, keratanases, reductases, oxidases, phenoloxidases, lipoxygenases, ligninases, pullulanases, tannases, pentosanases, malanases, $\beta$-glucanases, arabinosidases, hyaluronidase, chondroitinase, laccase, and amylases, or mixtures thereof. A typical combination may be a cocktail of conventional applicable enzymes like protease, lipase, cutinase and/or cellulase in conjunction with amylase. Enzymes can be used at their art-taught levels, for example at levels recommended by suppliers such as Novozymes and Genencor. Typical levels in the compositions are from about 0.0001% to about 5%. When enzymes are present, they can be used

at very low levels, e.g., from about 0.001% or lower; or they can be used in heavier-duty laundry detergent formulations at higher levels, e.g., about 0.1% and higher. In accordance with a preference of some consumers for "non-biological" detergents, the compositions may be either or both enzyme-containing and enzyme-free.

Dye Transfer Inhibiting Agents

The compositions may also include from about 0.0001%, from about 0.01%, from about 0.05% by weight of the compositions to about 10%, about 2%, or even about 1% by weight of the compositions of one or more dye transfer inhibiting agents such as polyvinylpyrrolidone polymers, polyamine N-oxide polymers, copolymers of N-vinylpyrrolidone and N-vinylimidazole, polyvinyloxazolidones and polyvinylimidazoles or mixtures thereof.

Chelant

The compositions may contain less than about 5%, or from about 0.01% to about 3% of a chelant such as citrates; nitrogen-containing, P-free aminocarboxylates such as EDDS, EDTA and DTPA; aminophosphonates such as diethylenetriamine pentamethylenephosphonic acid and, ethylenediamine tetramethylenephosphonic acid; nitrogen-free phosphonates e.g., HEDP; and nitrogen or oxygen containing, P-free carboxylate-free chelants such as compounds of the general class of certain macrocyclic N-ligands such as those known for use in bleach catalyst systems.

Bleach system

Bleach systems suitable for use herein contain one or more bleaching agents. Non-limiting examples of suitable bleaching agents include catalytic metal complexes; activated peroxygen sources; bleach activators; bleach boosters; photobleaches; bleaching enzymes; free radical initiators; H2O2; hypohalite bleaches; peroxygen sources, including perborate and/or percarbonate and combinations thereof. Suitable bleach activators include perhydrolyzable esters and perhydrolyzable imides such as, tetraacetyl ethylene diamine, octanoylcaprolactam, benzoyloxybenzenesulphonate, nonanoyloxybenzene-sulphonate, benzoylvalerolactam, dodecanoyloxybenzenesulphonate. Other bleaching agents include metal complexes of transitional metals with ligands of defined stability constants.

Stabilizer

The compositions may contain one or more stabilizers and thickeners. Any suitable level of stabilizer may be of use; exemplary levels include from about 0.01% to about 20%, from about 0.1% to about 10%, or from about 0.1% to about 3% by weight of the composition. Non-limiting examples of stabilizers suitable for use herein include crystalline, hydroxyl-containing stabilizing agents, trihydroxystearin, hydrogenated oil, or a variation thereof, and combinations thereof. In some aspects, the crystalline, hydroxyl-containing stabilizing agents may be water-insoluble wax-like substances, including fatty acid, fatty ester or fatty soap. In other aspects, the crystalline, hydroxyl-containing stabilizing agents may be derivatives of castor oil, such as hydrogenated castor oil derivatives, for example, castor wax. Other stabilizers include thickening stabilizers such as gums and other similar polysaccharides, for example gellan gum, carrageenan gum, and other known types of thickeners and rheological additives. Exemplary stabilizers in this class include gum-type polymers (e.g. xanthan gum), polyvinyl alcohol and derivatives thereof, cellulose and derivatives thereof including cellulose ethers and cellulose esters and tamarind gum (for example, comprising xyloglucan polymers), guar gum, locust bean gum (in some aspects comprising galactomannan polymers), and other industrial gums and polymers.

Silicones

Suitable silicones comprise Si-O moieties and may be selected from (a) non-functionalized siloxane polymers, (b) functionalized siloxane polymers, and combinations thereof. The molecular weight of the organosilicone is usually indicated by the reference to the viscosity of the material. The organosilicones may comprise a viscosity of from about 10 to about 2,000,000 centistokes at 25° C. Suitable organosilicones may have a viscosity of from about 10 to about 800,000 centistokes at 25° C.

TEST METHODS

It is understood the test methods disclosed in the TEST METHODS Section should be used to determine the respective values of the parameters described and claimed in the present application.

Procedure for Determination of Free Core Oil

The following method measures the amount of oil in the water phase. 1 mg/ml dibutyl phthalate (DBP)/hexane is used as an internal standard solution.

Weigh a little more than 250 mg DBP into a small beaker and then transfer the DBP into a 250 ml beaker. Fill the beaker with hexane to 250 ml.

Sample Prep: Weigh approximately 1.5-2 gram delivery particle slurry (40 drops) into a 20 ml scintillation vial, add 10 ml internal standard solution, and cap tightly. Shaking the vial vigorously several times over 30 minutes. Pipette the solution into an autosampler vial and analyze by gas chromatography (GC).

Instrumentation: HP5890 GC connected to HP Chem Station Software; Column: 5m x 0.32mm id with 1μm DB-1 liquid phase; Keep the temperature of the sample at 50°C for 1 minute then increase the temperature to 320 °C at the speed of 15 °C/min; Injector temperature is 275 °C; Detector temperature is 325 °C; Measurements have been performed with 2 ul injection.

Calculation: Add total peak area minus the area for the DBP for both the sample and calibration. The following equation is used to calculate mg of free core oil:

$$\frac{\text{Total area from sample}}{\text{Total area from calibration}} \times \text{mg of oil in calibration solution} = \text{mg of free oil}$$

The following equation is used to calculate % free core oil:

$$\frac{\text{mg of free core oil}}{\text{Sample wt. (mg)}} \times 100 = \text{\% free core oil in wet slurry}$$

The obtained values are shown in Table 2 as % free core.

Procedure for Determination of Benefit Agent Leakage

Obtain two delivery particle compositions encapsulating benefit agent, each weighs one gram. Add one delivery particle composition of 1 gram (Sample 1) to 99 grams of product matrix in which the delivery particle will be employed. Age the delivery particle containing product matrix (Sample 1) for applicable measurement time period at 35°C in a sealed glass jar. The other 1 gram delivery particle composition (Sample 2) is similarly aged.

After one week, use filtration to recover the delivery particle composition's delivery particles from the product matrix (Sample 1) and from the delivery particle composition (Sample 2). Treat each delivery particle composition with a solvent that will extract all the benefit agent from each sample. Inject the benefit agent containing solvent from each sample into a Gas Chromatograph and integrate the peak areas to determine the total quantity of benefit agent extracted from each sample.

Determine the percentage of benefit agent leakage by calculating the difference of the quantity of benefit agent extracted from Sample 2 and Sample 1, expressed as a percentage of the total quantity of benefit agent extracted from Sample 2, as represented in the equation below:

$$\text{Percentage of Benefit Agent Leakage} = \left(\frac{Sample\ 2 - Sample\ 1}{Sample\ 2}\right) \times 100$$

The values reported in Table 2 are % leakage (by weight) of the active material, used synonymously as benefit agent.

Procedure for Determination of Degradation

Degradation is determined by the "Organization for Economic Co-operation and Development (OECD) Guideline for Testing of Chemicals" 301B CO2 Evolution (Modified Sturm Test), adopted 17 July 1992. For ease of reference, this test method is referred to herein as test method OECD 301B.

EXAMPLES

Examples of the PAC/PBAE delivery particles are produced as set forth below. Table 1 sets forth the ingredient

list of key ingredients employed in the Examples. Table 2 presents data for Samples 1-8. The units are as indicated of the ingredients unless noted otherwise.

TABLE 1

| Tradename | Description | Manufacturer |
|---|---|---|
| SR230 | diethylene glycol diacrylate | Sartomer |
| SR351 | trifunctional trimethylolpropane triacrylate | Sartomer |
| SR415 | ethoxylated trimethylolpropane triacrylate | Sartomer |
| SR454 | ethoxylated trimethylolpropane triacrylate | Sartomer |
| Captex 355 | medium-chain triglyceride based on caprylic and capric acids | Abitec Corporation, Columbus, OH |
| SR444 | pentaerythritol triacrylate | Sartomer |
| DETA | diethylenene triamine | The DOW Chemical Company |
| Selvol 540 | polyvinyl alcohol | Sekisui Specialty Chemicals, Dallas, TX |
| CN975 | Hexafunctional urethane acrylate oligomer | Sartomer |
| CD9055 | carboxylic acid monofunctional acrylate monomer | Sartomer, Exton, PA |
| IPM | isopropyl myristate | Acme-Hardesty Co. |

**Example 1: Delivery particles containing perfume**

**Sample 1**

**[0264]** - Hybrid PAC/PBAE delivery particles that encapsulate a perfume are produced as follows. A first water phase was prepared by mixing 142.50g demineralized water and 80.00g of a 5wt% aqueous solution of Selvol 540 at room temperature.

A second water phase, which contains the PBAE prepolymer, was prepared by mixing 10.80g diethylenetriamine in 77.50g demineralized water at 35°C in a jacketed reactor. 22.40g diethylene glycol diacrylate was then added to the reactor and mixed at 35°C for 150 minutes. The second water phase was then cooled down to room temperature and added to the first water phase under mixing.

A first oil phase was prepared by mixing 22.41g perfume oil, 4.00g CN975, 0.048g TBAEMA and 0.048g CD9055 until a homogenous mixture was obtained.

A second oil phase was prepared by mixing 100.89g of the perfume oil, 100.89g isopropyl myristate, and 0.22g 2,2'-azobis(2-methylbutyronitrile) in a jacketed stainless-steel reactor. The reactor was held at 35 °C and the oil solution was mixed using an overhead mixer. A nitrogen blanket was applied to the reactor at a rate of 100 cc/min. The second oil composition was heated to 70°C over 45 minutes, held at 70 °C for 45 minutes, then cooled to 50 °C over 45 minutes. Once cooled, the first oil phase was added, and the combined oils are mixed for another 10 minutes.

The combined water phases are then added to the combined oil phase. High shear agitation was applied until the desired particle size was reached. The reactor was then mixed with a 3" diameter marine propeller blade. A third water phase which contains 20.70g trimethylolpropane triacrylate was then added to the above emulsion. The above emulsion was covered, and the temperature was increased to 75 °C over 60 minutes, held at 75 °C for 4 hours, increased to 95 °C over 60 minutes, and held at 95 °C for 6 hours. The batch was cooled to 25 °C over 90 minutes. The percentage of solids was measured to be 56.47 wt%. The volume weighted median particle size of the final slurry is 27.95 micron. The one week leakage of the slurry is 46.66%.

**Sample 2**

Hybrid PAC/PBAE delivery particles that encapsulate a perfume are produced as follows. A first water phase was prepared by mixing 142.50g demineralized water and 80.00g of a 5 wt% aqueous solution of Selvol 540 at room temperature.

A second water phase which contains the PBAE prepolymer was prepared by mixing 10.80g diethylenetriamine in 77.50g demineralized water at 25 °C in a in a jacketed reactor. 22.40g diethylene glycol diacrylate was then added

to the reactor and heated to 50 °C and then held at 50 °C for 150 minutes. The second water phase was then cooled down to room temperature and added to the first water phase under mixing.

A first oil phase was prepared by mixing 22.41g perfume oil, 4.00g CN975, 0.048g TBAEMA and 0.048g CD9055 until a homogenous mixture was obtained.

A second oil phase was prepared by mixing 100.89g of the perfume oil, 100.89g isopropyl myristate, and 0.22g 2,2'-azobis(2-methylbutyronitrile) in a jacketed stainless-steel reactor. The reactor was held at 35 °C and the oil solution was mixed using an overhead mixer. A nitrogen blanket was applied to the reactor at a rate of 100 cc/min. The second oil composition was heated to 70 °C over 45 minutes, held at 70 °C for 45 minutes, then cooled to 50 °C over 45 minutes. Once cooled, the first oil phase was added, and the combined oils are mixed for another 10 minutes at 50 °C.

The combined water phases are then added to the combined oil phase. High shear agitation was applied until the desired particle size was reached. The reactor was then mixed with a 3" diameter marine propeller blade. A third water phase which contains 20.70g trimethylolpropane triacrylate was then added to the above emulsion. The above emulsion was covered, and the temperature was increased to 75 °C over 60 minutes, held at 75 °C for 4 hours, increased to 95 °C over 60 minutes, and held at 95 °C for 6 hours. The batch was cooled to 25°C over 90 minutes. The percentage of solids was measured to be at 56.20wt%. The volume weighted median particle size of the final slurry is 24.19 micron. The one week leakage of the slurry is 43.58%.

## Sample 3

Hybrid PAC/PBAE delivery particles that encapsulate a perfume are produced as follows. A first water phase is prepared by mixing 108.0g demineralized water, 3.46g of a 5 wt% aqueous solution of Selvol 540 and 234.6g chitosan stock solution at room temperature. The chitosan stock solution is prepared in the following steps. Firstly 121.5 g chitosan is dissolved into 2578.5g demineralized water and 48.60g concentrated hydrochloric acid mixture at 25 °C. The dissolved chitosan mixture is then heated to 85 °C in 60 minutes and then held for 120 minutes before cools down to 25 °C in 90 minutes to obtain the chitosan stock solution.

An oil phase is prepared by mixing 66.59g perfume oil, 54.48g isopropyl myristate, 7.26g CN975 and 0.40g 2,2'-azobis(2-methylbutyronitrile) at room temperature until a homogenous mixture is obtained.

The oil phase is then added to the water phase at 70 °C under mixing. A nitrogen blanket is applied to the reactor at a rate of 100 cc/min. High shear agitation was then applied for a period of time until the desired particle size was reached. The reactor was then mixed with a 3" diameter marine propeller blade for 1 hour at 70 °C. A second water phase which contains 6.18g trimethylolpropane triacrylate was then added to the above emulsion. The above emulsion was covered, and the temperature was increased to 90 °C over 60 minutes, held at 90 °C for 8 hours. The batch was cooled to 25 °C over 90 minutes. The final slurry has volume weighted median particle size of 34.37 micron. The percentage of solids was measured at 32.72wt%. The one week leakage of the slurry was 18.02%.

## Sample 4

Hybrid PAC/PBAE delivery particles that encapsulate a perfume are produced as follows.

A first water phase was prepared by mixing 108.0g demineralized water, 21.90g of a 5 wt% aqueous solution of Selvol 540 and 234.6g chitosan stock solution at room temperature. The chitosan stock solution was prepared in the following steps. Firstly 121.5 g chitosan was dissolved into 2578.5g demineralized water and 48.60g concentrated hydrochloric acid mixture at 25 °C. The dissolved chitosan mixture was then heated to 85 °C in 60 minutes and then held for 120 minutes before cools down to 25 °C in 90 minutes to obtain the chitosan stock solution.

An oil phase was prepared by mixing 73.98g perfume oil, 60.53g isopropyl myristate, 2.46g CN975 and 0.14g 2,2'-azobis(2-methylbutyronitrile) at room temperature until a homogenous mixture was obtained.

The oil phase was then added to the water phase at 70 °C under mixing. A nitrogen blanket was applied to the reactor at a rate of 100 cc/min. High shear agitation was then applied for a period of time until the desired particle size was reached. The reactor was then mixed with a 3" diameter marine propeller blade for 1 hour at 70 °C. A second water phase which contains 10.26g trimethylolpropane triacrylate was then added to the above emulsion. The above emulsion was covered, and the temperature was increased to 90 °C over 60 minutes, held at 90 °C for 8 hours. The batch was cooled to 25 °C over 90 minutes. The final slurry has volume weighted median particle size of 29.87 micron. The percentage of solids was measured at 34.36wt%. The one week leakage of the slurry was 22.26%.

## Sample 5

Hybrid PAC/PBAE delivery particles that encapsulate a perfume are produced as follows. A first water phase was prepared by mixing 267.73g demineralized water and 51.00g of a 5 wt% aqueous solution of Selvol 540 at room temperature.

A first oil phase was prepared by mixing 12.80g perfume oil, 4.80g CN975, 0.06g TBAEMA and 0.06g CD9055 until a homogenous mixture was obtained.

A second oil phase was prepared by mixing 56.09g of the perfume oil, 100.89g Captex 355, and 0.27g 2,2'-azobis(2-methylbutyronitrile) in a jacketed stainless-steel reactor. The reactor was held at 35 °C and the oil solution was mixed using an overhead mixer. A nitrogen blanket was applied to the reactor at a rate of 100 cc/min. The second oil composition was heated to 70 °C over 45 minutes, held at 70 °C for 45 minutes, then cooled to 50 °C over 45 minutes. Once cooled, the first oil phase was added, and the combined oils mixed for another 10 minutes at 50 °C.

A third oil phase contains a PBAE prepolymer that was prepared by dissolving 22.86g diethylene glycol diacrylate into 32.00g perfume at room temperature and then heat to 50 °C in a jacketed reactor. 9.14g piperazine was then added to the above solution at 50 °C and then hold for 2 hours at 50 °C before cools to room temperature. The third oil phase was then added to the mixture of the first oil phase and the second oil phase at 50 °C and mixing for 10 minutes.
The first water phase was then added to the combined oil phase. High shear agitation was applied until the desired particle size was reached. The reactor was then mixed with a 3" diameter marine propeller blade. The above emulsion was covered, and the temperature was increased to 75 °C over 60 minutes, held at 75 °C for 4 hours, increased to 95 °C over 60 minutes, and held at 95 °C for 6 hours. The batch was cooled to 25 °C over 90 minutes. The percentage of solids was measured to be at 41.42wt%. The final slurry has volume weighted median particle size of 72.00 micron. The one week leakage of the slurry is 47.03%.

## Sample 6

Hybrid PAC/PBAE delivery particles that encapsulate a perfume are produced as follows. A first water phase was prepared by mixing 267.73g demineralized water and 51.00g of a 5 wt% aqueous solution of Selvol 540 at room temperature.

A first oil phase was prepared by mixing 12.80g perfume oil, 4.80g CN975, 0.06g TBAEMA and 0.06g CD9055 until a homogenous mixture was obtained.

A second oil phase was prepared by mixing 74.79g of the perfume oil, 100.89g Captex 355, and 0.27g 2,2'-azobis(2-methylbutyronitrile) in a jacketed stainless-steel reactor. The reactor was held at 35 °C and the oil solution was mixed using an overhead mixer. A nitrogen blanket was applied to the reactor at a rate of 100 cc/min. The second oil composition was heated to 70 °C over 45 minutes, held at 70 °C for 45 minutes, then cooled to 50 °C over 45 minutes. Once cooled, the first oil phase was added, and the combined oils mixed for another 10 minutes at 50 °C.

A third oil phase contains a PBAE prepolymer that was prepared by dissolving 9.50g diethylene glycol diacrylate into 13.30g perfume at room temperature and then heat to 50 °C in a jacketed reactor. 3.80g piperazine was then added to the above solution at 50 °C and then hold for 2 hours at 50 °C before cools to room temperature. The third oil phase was then added to the mixture of the first oil phase and the second oil phase at 50 °C and mixing for 10 minutes.

The first water phase was then added to the combined oil phase. High shear agitation was applied until the desired particle size was reached. The reactor was then mixed with a 3" diameter marine propeller blade. The above emulsion was covered, and the temperature was increased to 75 °C over 60 minutes, held at 75 °C for 4 hours, increased to 95 °C over 60 minutes, and held at 95 °C for 6 hours. The batch was cooled to 25 °C over 90 minutes. The percentage of solids was measured to be at 40.55wt%. The final slurry has volume weighted median particle size of 17.41 micron. The one week leakage of the slurry was 48.18%.

## Sample 7

Hybrid PAC/PBAE delivery particles that encapsulate a perfume are produced as follows. A water phase was prepared by dissolving 15.05g gelatin (Type B, 225 bloom) into 210.00g demineralized water at 70 °C.

An oil phase was prepared by mixing 86.31g perfume oil, 70.62g isopropyl myristate, 2.87g CN975 and 0.16g 2,2'-azobis(2-methylbutyronitrile) at room temperature until a homogenous mixture was obtained.
The oil phase was then added to the water phase at 70 °C under mixing. High shear agitation was then applied for a period of time until the desired particle size was reached. A nitrogen blanket was applied to the reactor at a rate of 100 cc/min. The reactor was then mixed with a 3" diameter marine propeller blade for 1 hour at 70 °C. A second water phase which contains 3.64g trimethylolpropane triacrylate and 5.14g tetra(ethylene glycol) diacrylate was then added to the above emulsion. The above emulsion was covered, and the temperature was increased to 90 °C over 60 minutes, held at 90 °C for 8 hours. The batch was cooled to 25 °C over 90 minutes. The final slurry has volume weighted median particle size of 39.09 micron. The percentage of solids was measured at 52.71wt%. The one week leakage of the slurry was 45.60%.

**Sample 8**

Hybrid PAC/PBAE delivery particles that encapsulate a perfume are produced as follows.

A first water phase was prepared by mixing 85.50g demineralized water and 48.00g of a 5wt% aqueous solution of Selvol 540 at room temperature.

A second water phase, which contains the PBAE prepolymer, was prepared by mixing 6.48g diethylenetriamine in 46.50g demineralized water at 35°C in a jacketed reactor. 13.44g diethylene glycol diacrylate was then added to the reactor and mixed at 35°C for 150 minutes. The second water phase was then cooled down to room temperature and added to the first water phase under mixing.

An oil phase was prepared by mixing 73.98g perfume oil, 60.53g isopropyl myristate, 2.46g CN975 and 0.14g 2,2'-azobis(2-methylbutyronitrile) at room temperature until a homogenous mixture was obtained.
The oil phase was then added to the combined water phase at 70 °C under mixing. A nitrogen blanket was applied to the reactor at a rate of 100 cc/min. High shear agitation was then applied for a period of time until the desired particle size was reached. The reactor was then mixed with a 3" diameter marine propeller blade for 1 hour at 70 °C. A second water phase which contains 10.26g trimethylolpropane triacrylate was then added to the above emulsion. The above emulsion was covered, and the temperature was increased to 90 °C over 60 minutes, held at 90 °C for 8 hours. The batch was cooled to 25 °C over 90 minutes. The final slurry has volume weighted median particle size of 36.87 micron. The percentage of solids was measured at 61.03wt%. The one week leakage of the slurry was 59.10%.

Example 2: Liquid Fabric Softener Comprising Delivery Particles

Liquid Fabrice Softener comprising Delivery Particles was prepared as shown in TABLE 2.

A fabric softener composition was prepared according to WO2018/170356. The fabric softener composition was finished by adding the delivery particle slurry using an IKA Ultra Turrax (dispersing element 8G) operated at 10 000 rpm for 1 minute, as shown below in TABLE 2.

TABLE 2

| | Sample 1A | Sample 3A | Sample 4A | Sample 7A |
|---|---|---|---|---|
| | Weight % | | | |
| Deionized water | To balance | To balance | To balance | To balance |
| NaHEDP | 0.007 | 0.007 | 0.007 | 0.007 |
| Formic acid | 0.045 | 0.045 | 0.045 | 0.045 |
| HCl | 0.001 | 0.001 | 0.001 | 0.001 |
| Preservative[a] | 0.023 | 0.023 | 0.023 | 0.023 |
| FSA[b] | 9.19 | 5 | 11 | 9.19 |
| Antifoam[c] | 0.101 | 0.101 | 0.101 | 0.101 |
| Coconut oil | 0.31 | 0.31 | 0.31 | 0.31 |
| Isopropanol | 0.94 | 0.79 | 1.05 | 0.94 |
| $CaCl_2$ | 0.008 | 0.008 | 0.008 | 0.008 |
| Perfume | 0.4 | | 0.6 | |
| Perfume via delivery particles from Sample 1 | 0.25 | | | |
| Perfume via delivery particles from Sample 3 | | 0.4 | | |
| Perfume via delivery particles from Sample 4 | | | 0. 23 | |
| Perfume via delivery particles from Sample 7 | | | | 0.4 |

(continued)

| | Sample 1A | Sample 3A | Sample 4A | Sample 7A |
|---|---|---|---|---|
| | Weight % | | | |
| Deionized water | To balance | To balance | To balance | To balance |
| Cationic polymer[d] | 0.3 | 0.3 | 0.3 | 0.3 |

[a]Proxel GXL, 20% aqueous dipropylene glycol solution of 1,2-benzisothiazolin-3-one, supplied by Lonza. This material was part of the dispersion that was made and was not added at another point in the process.
[b]DEEDMAC: diethyl-ester-dimethyl-ammonium-chloride
[c]MP10®, supplied by Dow Corning, 8% activity
[d]Rheovis® CDE, cationic polymeric acrylate thickener supplied by BASF

Example 3: Liquid Laundry Detergent Comprising Delivery Particles

Liquid Laundry Detergent Compositions comprising the Delivery Particles were prepared and the Delivery Particles tested for leakage, as described in the TEST METHODS Section, and as shown in TABLE 3 below.

TABLE 3

| | Sample 1B | Sample 2B | Sample 5B | | Sample 6B |
|---|---|---|---|---|---|
| Ingredient: | %wt | | | | |
| C12-45 alkyl-7-ethoxylated | 2.34 | | | | |
| C12-14 alkyl-7-ethoxylated | 0.2 | | | | |
| Monoethanolamine: $C_{12-14}$ EO·3·$SO_3H$ | 0.5 | | | | |
| Linear alkyl benzene sulfonic acid | 4 | | | | |
| sodium hydroxide | 1.9 | | | | |
| sodium cumene sulfonate | 0.18 | | | | |
| citric acid | 1.4 | | | | |
| C12-18 Fatty acid | 1.1 | | | | |
| Solvents (1,2-Propanediol, Ethanol) | 1.1 | | | | |
| Chelants | 0.2 | | | | |
| Soil suspending alkoxylated polyalkylenimine polymer[a] | 0.68 | | | | |
| Minors (stabilizers, preservatives...) | 1 | | | | |
| Hydrogenated castor oil | 0.2 | | | | |
| Perfume via delivery particles from Sample 6 | 0.5 | | | | |
| Perfume via delivery particles from Sample 7 | | 0.5 | | | |
| Perfume via delivery particles from Sample 8 | | | 0.5 | | |
| Perfume via delivery particles from Sample 9 | | | | | 0.5 |
| water | up to 100 | | | | |
| Leakage | | 46.66 | 43.58 | 47.03 | 48.18 |

[a]600g/mol molecular weight polyethylenimine core with 24 ethoxylate groups per -NH and 16 propoxylate groups per -NH. Available from BASF (Ludwigshafen, Germany)

The dimensions and values disclosed herein are not to be understood as being strictly limited to the exact numerical values recited. Instead, unless otherwise specified, each such dimension is intended to mean both the recited

value and a functionally equivalent range surrounding that value. For example, a dimension disclosed as "40 mm" is intended to mean "about 40 mm". Unless otherwise indicated, measurements are on the basis of weight, and in the metric system.

Every document cited herein, including any cross referenced or related patent or application and any patent application or patent to which this application claims priority or benefit thereof, is hereby incorporated herein by reference in its entirety unless expressly excluded or otherwise limited. The citation of any document is not an admission that it is prior art with respect to any invention disclosed or claimed herein or that it alone, or in any combination with any other reference or references, teaches, suggests or discloses any such invention. Further, to the extent that any meaning or definition of a term in this document conflicts with any meaning or definition of the same term in a document incorporated by reference, the meaning or definition assigned to that term in this document shall govern.

While particular embodiments of the present invention have been illustrated and described, it would be obvious to those skilled in the art that various other changes and modifications can be made without departing from the spirit and scope of the invention. It is therefore intended to cover in the appended claims all such changes and modifications that are within the scope of this invention.

## Claims

1. A consumer product composition comprising a treatment adjunct and a polyacrylate and poly(beta-amino esters) (PAC/PBAE) delivery particle, wherein the delivery particle comprises:

   a core; and
   a shell comprising PAC, hybrid PAC/PBAE, and PBAE, wherein the shell is derived from i) 5% to 90% of a preformed PBAE prepolymer, or a polyamine, or a mixture of a first water-soluble or dispersible multifunctional acrylate and a polyamine, ii) 0.1% to 90% of a multifunctional (meth)acrylate monomer, iii) at least one oil soluble or dispersible thermal free radical initiator, iv) 0.1% to 90% of a second water-soluble or dispersible multifunctional acrylate, and v) 0% to 10% of a monofunctional acidic or basic (meth)acrylate monomer, by weight of the shell.

2. The consumer product composition according to claim 1, wherein the preformed PBAE prepolymer contains free amino moieties reactive with the multifunctional (meth)acrylate via Aza-Michael Addition reaction.

3. The consumer product composition according to claim 1 or 2, wherein the preformed PBAE prepolymer contains free (meth)acrylate moieties reactive with the multifunctional (meth)acrylate via free radical polymerization.

4. The consumer product composition according to any of claims 1 to 3, wherein the preformed PBAE prepolymer is derived from a first water-soluble or dispersible multifunctional acrylate and a multifunctional amine, wherein a molar ratio of the first multifunctional acrylate to the multifunctional amine is in a range from about 100:1 to about 1:100, preferably in a range from about 10:1 to about 1:10, more preferably in a range from about 2:1 to about 1:2.

5. The consumer product composition according to any of the previous claims, wherein the shell has a contiguous covalently-linked shell structure comprising hybrid PAC/PBAE.

6. The consumer product composition according to any of the previous claims, wherein the shell has a dual shell structure comprising an inner shell and an outer shell, a composition of the inner shell comprises hybrid PAC/PBAE, a composition of the outer shell comprises PBAE, wherein the composition of the outer shell crosslinks or deposits to the composition of the inner shell via covalent bond.

7. The consumer product composition according to any of the previous claims, wherein the shell has a multi-shell structure comprising an inner shell, a transitional shell and an outer shell, a composition of the inner shell comprises PAC, a composition of the transitional shell comprises hybrid PAC/PBAE, a composition of the outer shell comprises PBAE, and the composition of each shell crosslinks or deposits to the composition of an adjacent shell.

8. The consumer product composition according to any of the previous claims, wherein the multifunctional (meth)acrylate is selected from group consisting of tri-functional (meth)acrylate, tetra-functional (meth)acrylate, penta-functional (meth)acrylate, hexafunctional (meth)acrylate, hepta-functional (meth)acrylate, and mixtures thereof.

9. The consumer product composition according to any of the previous claims, wherein the multifunctional (meth)acrylate comprises a multifunctional aromatic urethane acrylate.

10. The consumer product composition according to any of the previous claims, wherein the first and the second water-soluble or dispersible multifunctional acrylate is selected from diethylene glycol diacrylate, triethylene glycol diacrylate, tetraethylene glycol diacrylate, poly(ethylene glycol) diacrylate, trifunctional trimethylolpropane triacrylate, ethoxylated trimethylolpropane triacrylate independently or a combination thereof.

11. The consumer product composition according to any of the previous claims, wherein the basic (meth)acrylate monomer is selected from the group consisting of ethylaminoethyl acrylate, ethylaminoethyl methacrylate, aminoethyl acrylate, aminoethyl methacrylate, tertiarybutyl aminoethyl acrylate, tertiarybutyl aminoethyl methacrylate, diethylamino acrylate, diethylamino methacrylate, diethylaminoethyl acrylate diethylaminoethyl methacrylate, dimethylaminoethyl acrylate and dimethylaminoethyl methacrylate, and the acidic (meth)acrylate monomer is selected from the group consisting of 2-carboxyethyl acrylate, 2-carboxyethyl methacrylate, 2-carboxypropyl acrylate, 2-carboxypropyl methacrylate, carboxyoctyl acrylate, carboxyoctyl methacrylate, 2-acryloyloxybenzoic acid, 3-acryloyloxybenzoic acid, 4-acryloyloxybenzoic acid, 2-methacryloyloxybenzoic acid, 3-methacryloyloxybenzoic acid, and 4-methacryloyloxybenzoic acid, 4-acryloyloxyphenylacetic acid, and 4-methacryloyloxyphenylacetic acid.

12. The consumer product composition according to any of the previous claims, wherein the oil soluble or dispersible thermal free radical initiator is an azo-based initiator.

13. The consumer product composition according to any of the previous claims, wherein the polyamine is selected from aminoethylpiperazine, N,N'-Bis-(2-aminoethyl)piperazine), piperazine, diethylenetriamine, ethylenediamine, triethylenetetramine, pentaethylenehexamine, polyethylenimine, chitosan, chitin, gelatin, arginine, lysine, ornithine, nisin, histidine, and mixtures thereof.

14. The consumer product composition according to any of the previous claims, wherein the delivery particle has a leakage of below about 50%, preferably about 30%, as determined by the Leakage Test described in the TEST METHODS Section.

15. A method of treating a surface, wherein the method comprises the step of contacting the surface with a consumer product composition according to any of the previous claims, optionally in the presence of water.

European Patent Office

# EUROPEAN SEARCH REPORT

Application Number
EP 21 20 8928

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2008/127423 A2 (CORNELL RES FOUNDATION INC [US]; MCQUADE D TYLER [US] ET AL.) 23 October 2008 (2008-10-23) * claim 5 * ----- | 1-14 | INV. B01J13/14 A01N25/28 A61K8/11 A61K9/50 B01J13/16 B01J13/22 C09B67/02 C11D3/50 D06M23/12 F28D20/02 |
| X,D | WO 2010/079468 A2 (PROCTER & GAMBLE [US]; DIHORA JITEN ODHAVJI [US] ET AL.) 15 July 2010 (2010-07-15) * claims 1, 24-25 * ----- | 15 | |
| A | US 2005/244504 A1 (LITTLE STEVEN R [US] ET AL) 3 November 2005 (2005-11-03) * the whole document * ----- | 1-15 | |

TECHNICAL FIELDS SEARCHED (IPC)

B01J
A01N
C09B
C11D
A61Q
D06Q
A61K
F28F
D06M
F28D

The present search report has been drawn up for all claims

1

| Place of search | Date of completion of the search | Examiner |
|---|---|---|
| The Hague | 28 March 2022 | Tarallo, Anthony |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.........................................................
& : member of the same patent family, corresponding document

EPO FORM 1503 03.82 (P04C01)

# ANNEX TO THE EUROPEAN SEARCH REPORT ON EUROPEAN PATENT APPLICATION NO.

EP 21 20 8928

This annex lists the patent family members relating to the patent documents cited in the above-mentioned European search report. The members are as contained in the European Patent Office EDP file on
The European Patent Office is in no way liable for these particulars which are merely given for the purpose of information.

28-03-2022

| Patent document cited in search report | Publication date | Patent family member(s) | Publication date |
|---|---|---|---|
| WO 2008127423 A2 | 23-10-2008 | NONE | |
| WO 2010079468 A2 | 15-07-2010 | CA 2795617 A1 | 15-07-2010 |
| | | CN 102892492 A | 23-01-2013 |
| | | EP 2563508 A2 | 06-03-2013 |
| | | ES 2746202 T3 | 05-03-2020 |
| | | JP 6212079 B2 | 11-10-2017 |
| | | JP 2013525564 A | 20-06-2013 |
| | | JP 2016011425 A | 21-01-2016 |
| | | KR 20130000417 A | 02-01-2013 |
| | | MX 354698 B | 15-03-2018 |
| | | PL 2563508 T3 | 31-03-2020 |
| | | RU 2012142728 A | 10-06-2014 |
| | | US 2011268802 A1 | 03-11-2011 |
| | | US 2015071977 A1 | 12-03-2015 |
| | | US 2019142714 A1 | 16-05-2019 |
| | | WO 2010079468 A2 | 15-07-2010 |
| US 2005244504 A1 | 03-11-2005 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 7427394 B2
- US 8808681 B2
- US 20190125874
- US 6869923 B1
- US 20110268802
- US 10415000 B2
- US 10485739 B2
- US 20150030557
- WO 2006117702 A
- US 20170296440 A, Gross
- US 20080193761 A
- US 6649155 B
- US 20080317698
- US 20080206355
- US 2486921 A
- US 2486922 A
- US 2396278 A
- US 5104646 A
- US 5106609 A
- US 3929678 A
- US 2658072 A
- US 2438091 A
- US 2528378 A
- US 4741855 A
- US 20120009285
- US 157665
- US 20040092415 A1, Focht
- US 5487884 A, Bisset
- US 5681852 A
- US 5652228 A, Bisset
- US 6395691 B, Liang Sheng Tsaur
- US 6645511 B, Aronson
- US 6759376 B, Zhang
- US 6780826 B, Zhang
- US 20060182699 A, Taylor
- US 5624666 A
- US 5100660 A
- US 4849484 A
- US 4835206 A
- US 4628078 A
- US 4599379 A
- EP 228868 A
- US 6967027 B, Heux
- US 5207826 A, Westland
- US 4487634 A, Turbak
- US 4373702 A, Turbak
- US 4863565 A, Johnson
- US 20070027108 A, Yang
- WO 2018170356 A


### Non-patent literature cited in the description


- **STEFFEN ARCTANDER.** Perfume and Flavor Chemicals. Allured Pub. Co, 1994, vol. I,II
- **MILLER, P. M. ; LAMPARSKY, D.** Perfumes: Art, Science and Technology. Blackie Academic and Professional, 1994
- McCutcheon's, Emulsifiers and Detergents. M. C. Publishing Co, 1989
- McCutcheon's: Detergents and Emulsifiers. Allured Publishing Corporation, 1947
- McCutcheon's, Functional Materials. Allured Publishing Corporation, 1973
- McCutcheon's, Detergents and Emulsifiers. allured Publishing Corporation, 1986
- McCutcheon's, Functional Materials. 1992